# EUROPEAN PATENT APPLICATION

(11) **EP 3 884 990 A1**
(43) Date of publication of application: **29.09.2021**
(21) Application number: 19886911.7
(22) Date of filing: 20.11.2019
(51) Int. Cl.: A61M 39/10, A61M 39/26

(54) **MALE CONNECTOR**

(30) Priority: 20.11.2018 JP 2018217356; 20.08.2019 JP 2019150221
(71) Applicant: Nipro Corporation, Osaka-shi, Osaka 531-8510 (JP)
(72) Inventor: IMAI, Kenta, Osaka-shi, Osaka 531-8510 (JP); MATSUMOTO, Kazuki, Osaka-shi, Osaka 531-8510 (JP)
(74) Representative: Bertsch, Florian Oliver
(86) International application number: PCT/JP2019/045517
(87) International publication number: WO 2020/105690

(57) **Abstract**

A male connector 10 includes a side hole needle 14 that has an inner hole 16 opened sideways at a tip of a hollow needle and places a fluid flow path 106 in a communicating state by insertion of the side hole needle 14 into a female connector 86 as a connection partner. The male connector 10 has an elastic cover valve body 74 that covers a lateral opening 12 of the inner hole 16 at the tip of the side hole needle 14 such that the cover valve body 74 is axially movable relative to the side hole needle 14, and has a positioning means 112 that elastically positions the cover valve body 74 to the tip of the side hole needle 14 while allowing movement of the cover valve body 74 to a proximal end side of the side hole needle 14. The male connector 10 has a rigid restraining member 56 that covers an outer periphery of the cover valve body 74.

## Description

### TECHNICAL FIELD

The present invention relates to a male connector connected to a female connector in a fluid flow path used in the medical field.

### BACKGROUND ART

In the medical field, fluid channels such as infusion lines and blood transfusion lines have been used to administer or collect liquids such as water, electrolytes, chemicals such as anticancer agents, nutrients, blood, and contrast media. In such a fluid flow path, in order to connect another fluid flow path as needed, a male connector is provided at the end of one flow path, a female connector is provided at the end of the other flow path, and the male connector and the female connector are made to connect to each other.

Specifically, for example, a male connector provided with a hollow needle or a male luer is provided at the end of a flow path extending from a container such as a syringe containing a drug solution such as an anticancer drug, while a female connector is provided at the end of the flow path such as a catheter inserted into a patient. This makes it possible to realize the connection and disconnection of the two fluid flow paths by attaching and detaching the male connector to the female connector.

In the male connector, it is preferable that the opening of the flow path is closed in order to prevent exposure or leakage of the chemical solution or the like to the outside when the connector is not connected to the female connector. One example of such a male connector is shown in Japanese Unexamined Patent Publication No. JP-A-2013-121525 (Patent Document 1). In the embodiment described in FIGS. 1 to 9 of Patent Document 1, a hollow valve member (16) is positioned on the inner peripheral side of the male luer in the male connector (10). As shown in FIG. 8 of Patent Document 1, for example, when not connected to the female connector (92), the tip of the valve member (16) having a window (54) that opens into the peripheral wall is fitted into the tip opening of the male luer (22), whereby shutting off the opening of the fluid flow path of the male connector. On the other hand, as shown in FIG. 9 of Patent Document 1, when connecting to the female connector (92), the valve member (16) is pushed behind the male luer (22) by the female connector (92), so that the lumen of the valve member (16) is opened through the window (54) and the opening at the tip of the male luer (22), whereby the opening of the fluid flow path of the male connector is held in communication with the fluid flow path of the female connector (92).

Further, International Publication No. WO2018/056465 (Patent Document 2) also shows a male connector in which the opening of the flow path is closed when the connector is not connected to the female connector. That is, in the embodiment described in FIGS. 1 to 5 of Patent Document 2, the convex portion (22) of the housing body (20a) and the concave portion (31) of the moving body (30a) are mated together when the male connector (1a) is not connected to the female connector (2a). The moving body (30a) is prevented from moving in the axial direction with respect to the housing body (20a), and the opening (42) of the flow path (first flow path 41) of the male connector (1a) is closed by the valve body (50a). The female connector (2a) is connected to the moving body (30a) of the male connector (1a), and the female connector (2a) and the moving body (30a) are moved to the proximal end side with respect to the housing body (20a). By moving, the engagement between the convex portion (22) and the concave portion (31) is released. Then, by further moving the female connector (2a) and the moving body (30a) to the proximal end side, the tip (44) of the flow path tube member (40a) constituting the flow path (first flow path 41) of the male connector (1a) is inserted into the female connector (2a), while penetrating the valve body (50a) and the elastic valve body (70). As a result, the flow path (first flow path 41) of the male connector (1a) and the flow path (second flow path 66) of the female connector (2a) are communicated with each other.

### BACKGROUND ART DOCUMENT

### PATENT DOCUMENT

Patent Document 1: JP-A-2013-121525
Patent Document 2: WO2018/056465

### SUMMARY OF THE INVENTION

### PROBLEM THE INVENTION ATTEMPTS TO SOLVE

However, in the male connector (10) described in FIGS. 1 to 9 of Patent Document 1, the window (54) of the valve member (16) is not directly closed when the male luer is not connected to the female luer. In addition, there is a problem that it is difficult to secure the sealing property between the tip of the valve member (16) and the fitting portion to the male luer (22). Thus, further improvements are required for prevention of exposure and leakage of chemicals and the like in the connector structure.

Further, in the embodiment shown in FIGS. 1 to 5 of Patent Document 2, in a series of operations for connecting the male connector (1a) and the female connector (2a), the female connector (2a) and the moving body (30a) are moved to the proximal end side with respect to the housing main body (20a), whereby the peripheral wall of the housing body (20a) having the convex portion (22) is elastically deformed to the outer peripheral side, and the convex portion (22) and the concave portion (31) are disengaged from each other. Therefore, in addition to the operating force for simply moving the female connector (2a) and the moving body (30a) toward the proximal end side, another operating force for detaching the convex portion (22) from the recess (31) by elastically deforming the peripheral wall of the housing body (20a) toward the outer peripheral side. Thus, the operability may be deteriorated. Also, the elastically deformed portion of the housing body (20a) may be gripped by hand, possibly making it less likely to come off the detachment of the convex portion (22) from the recess (31). Furthermore, the stability and reliability of operation may not be sufficiently ensured.

Furthermore, the disengagement between the convex portion (22) and the concave portion (31) and the movement of the female connector (2a) and the moving body (30a) toward the proximal end side are performed as a series of operations. Therefore, for example, if the engaging force between the convex portion (22) and the concave portion (31) is set to be large, the operating force required to disengage the engagement is also increased. This may interfere with the operation of the connection between the male connector (1a) and the female connector (2a). On the other hand, when the engaging force between the convex portion (22) and the concave portion (31) is set small, the engagement between the convex portion (22) and the concave portion (31) may be unintentionally released. This may cause the moving of the moving body (30a) to the proximal side, and the opening of the flow path (first flow path 41) of the male connector (1a) upon the male connector (1a) being unconnected to the female connector (2a). That is, by performing the disengagement between the convex portion (22) and the concave portion (31) and the movement of the female connector (2a) and the moving body (30a) toward the proximal end side as a series of operations, it was difficult to achieve both improved stability and improved movement operability.

It is an object of the present invention to provide a male connector having a new structure capable of solving at least one of the problems inherent in a male connector having a conventional structure. Namely, the male connector according to the present invention may achieve at least one of the followings: the flow path can be stably closed when the female connector is not connected; the stability and reliability of the connection operation to the female connector can be improved; stable closing of the flow path at the time of non-connection with the female connector as well as improvement of operability of the connection operation to the female connector can be achieved at the same time; the blocking performance of the flow path from the external space at the time of connection with the female connector can be improved; and an exposure of a chemical solution can be more reliably prevented.

### MEANS FOR SOLVING THE PROBLEM

Hereinafter, preferred embodiments for grasping the present invention will be described, but each of the embodiments described below is described as an example, and can be adopted in an appropriate combination with each other. The plurality of components described in the each embodiment can be recognized and adopted independently as much as possible, and can be appropriately adopted in combination with any of the components described in another embodiment. Thereby, in the present invention, various other aspects can be realized without being limited to the aspects described below.

A first preferred embodiment provides a male connector comprising: a side hole needle having a hollow needle with an inner hole and an opening through which the inner hole is opened sideways at a tip of the hollow needle, the side hole needle being configured to be inserted into a female connector as a connection partner so as to connect a fluid flow path thereof with that of the female connector; an elastic cover valve body provided so as to be movable in an axial direction with respect to the side hole needle, the elastic cover valve body being configured to cover the opening of the inner hole at the tip of the side hole needle; a positioning means for elastically positioning the cover valve body to the tip of the side hole needle while allowing the cover valve body to move to a proximal end side of the side hole needle; and a rigid restraining member that covers an outer periphery of the cover valve body.

According to the male connector of the present preferred embodiment, since the opening of the side hole needle is covered with an elastic cover valve body and closed, the closing of the male connector when not connected to the female connector can be stably realized. Further, the rigid restraining member that covers the outer periphery of the cover valve body stabilizes the deformation mode of the cover valve body, and the cover valve body can improve the closing performance of the side hole needle.

A second preferred embodiment provides the male connector according to the first preferred embodiment, wherein the restraining member includes: a proximal end side protrusion extending toward a proximal end side of the side hole needle; and a guide portion provided at the proximal end side protrusion to guide an axial movement of the restraining member with respect to a male connector housing.

According to the male connector of the present preferred embodiment, the restraining member can be moved more smoothly in the axial direction, and the deformation mode of the cover valve body can be stabilized.

A third preferred embodiment provides the male connector according to the first or second preferred embodiment, further comprising a male connector housing having a tubular peripheral wall portion that covers an outer periphery of the restraining member.

According to the male connector of the present preferred embodiment, for example, the positioning means for positioning the cover valve body can be provided inside the tubular peripheral wall portion, and the positioning of the cover valve body can be prevented from being unintentionally released.

A fourth preferred embodiment provides the male connector according to the third preferred embodiment, wherein the tubular peripheral wall portion has a structure separate from a needle hub of the side hole needle.

According to the male connector of the present preferred embodiment, for example, by assembling the needle hub with the restraining member arranged inside the tubular peripheral wall portion, a structure in which the outer peripheral side of the restraining member is covered by the tubular peripheral wall portion can be easily realized.

A fifth preferred embodiment provides the male connector according to the third or fourth preferred embodiment, wherein the tubular peripheral wall portion has a tubular cover portion protruding toward a tip side of the side hole needle and having a diameter larger than that of the tubular peripheral wall portion.

According to the male connector of the present preferred embodiment, since the cover portion has a sufficiently large diameter, contact with the female connector is prevented even when the female connector is brought close to each other, and good operability is exhibited. In addition, since the cover portion protrudes toward the tip side with a sufficient length, the outer peripheral side of the cover valve body can be more reliably covered with the cover portion. This makes it possible to prevent leakage or exposure of the drug solution due to an unintentional contact of a user with the cover valve body.

A sixth preferred embodiment provides the male connector according to any one of the first to fifth preferred embodiments, further comprising a connecting portion configured to be detachably connected to the female connector with the cover valve body being abutted and superposed on a valve member of the female connector, wherein the side hole needle is insert-able and removable from the cover valve body and the valve member of the female connector under a contact state of the cover valve body with the valve member of the female connector by the connecting portion.

According to the male connector of the present preferred embodiment, the side hole needle can be inserted and removed from the cover valve body and the valve member of the female connector in a state of being overlapped with each other, thereby further preventing exposure and leakage of chemicals and the like.

A seventh preferred embodiment provides a male connector comprising: a tubular body having an inner hole and an opening through which the inner hole is opened at a tip of the tubular body, the tip of the tubular body being configured to be inserted into a female connector as a connection partner so as to connect a fluid flow path thereof with that of the female connector; an elastic cover valve body configured to cover the opening of the tip of the tubular body; a holding member holding the cover valve body and being movable in an axial direction of the tubular body; and a releasable movement restriction mechanism for restricting a movement of the holding member holding the cover valve body in the axial direction of the tubular body so as to hold the opening of the tip of the tubular body in a sealed state by the cover valve body.

According to the male connector of the present preferred embodiment, the stability of the sealed state by the cover valve body at the opening of the tip of the tubular body can be improved by restricting the movement of the holding member that holds the cover valve body.

An eighth preferred embodiment provides a male connector comprising: a tubular body having an inner hole and an opening through which the inner hole is opened at a tip of the tubular body; and an elastic cover valve body configured to cover the opening of the tip of the tubular body, wherein the tip of the tubular body is configured to be projected out from the cover valve body to be inserted into a female connector as a connection partner so as to connect a fluid flow path thereof with that of the female connector, a tip surface of the cover valve body has a centrally protruding shape that protrude axially outwardly from an outer periphery to a central, a central portion of the tip surface of the cover valve body is a curved tip surface that is convex toward the tip, and a region that spreads out from the central portion toward the outer periphery is an inverted curved inclined surface that becomes concave toward the tip.

According to the male connector of the present preferred embodiment, the region extending from the central portion to the outer periphery of the tip surface of the cover valve body is an inverted curved inclined surface. Therefore, the female connector is connected to the female connector with a gap generated between the overlapping surfaces with the surface of the valve body on the side being reduced, for example, in a close contact state. As a result, for example, it becomes possible to suppress the exudation or leakage of the chemical solution or the like into the gap.

By the way, in the male connector targeted by the present invention, it has been recognized that improving the exposure prevention performance of the chemical solution is one of the important issues. That is, for example, the heat effect during sterilization (especially because heat is applied in the initial state where the needle tip is stuck in the cover valve body) may reduce the exposure prevention performance of the cover valve body. Repeated needle penetration through the cover valve body due to multiple uses, for example, may make it easier for the slit to open, or may cause a deformation of the valve body by being pulled by the needle or due to frictional resistance, etc. These possibilities may reduce the exposure prevention performance of the cover valve body.

In particular, as a result of the extended examination by the present inventor, a new recognition has been obtained that the exposure prevention performance on the proximal end side of the cover valve body is a new point of interest (one of the new subject matters). For example, (i) In the conventional structure, since a single or radial slit is adopted in the cover valve body, a mechanism in which gaps are likely to occur on both sides in the slit direction when inserting a tubular body such as a side hole needle: (ii) the cover valve body is deformed to extend in the axial direction due to the compressive force applied in the radial direction; and (iii) Due to the fact that the coil spring is pressed against the front end portion of the cover valve body, the rear end portion of the valve body tends to be locally distorted. For the above reasons, the rear end portion of the cover valve body tends to cause expose of the chemical solution.

In addition, when the side hole needle is removed from the valve body, the inner surface of the slit of the cover valve body is pulled backward by the friction of the tubular body such as the side hole needle. Therefore, the compressive force in the radial direction tends to decrease and the rear end of the slit is easily deformed in the direction of opening. The present inventors found a new point of view that the risk of exposure of the drug solution from the rear end of the valve body is further increased. By paying attention to that point, the present inventors are able to achieve technical effects such as an improved exposure prevention performance, or the like.

A ninth preferred embodiment provides a male connector comprising: a side hole needle having a hollow needle with an inner hole and an opening through which the inner hole is opened sideways at a tip of the hollow needle, the side hole needle being configured to be inserted into a female connector as a connection partner so as to connect a fluid flow path thereof with that of the female connector; an elastic cover valve body provided so as to be movable in an axial direction with respect to the side hole needle, the elastic cover valve body being configured to cover the opening of the inner hole at a tip of the side hole needle; and a pin hole provided on a central axis of the cover valve body on which the side hole needle is inserted, the pin hole having a diameter at least at a base side smaller than that of the side hole needle.

According to the male connector of the present preferred embodiment, in the cover valve body, for example, a pin hole having a diameter smaller than that of the side hole needle is provided instead of the slit. Therefore, when the side hole needle is inserted into or removed from the cover valve body, the gap between the cover valve body and the side hole needle can be reduced in comparison with the case of the slit, thereby more reliably preventing leakage and exposure of the chemical solution.

A tenth preferred embodiment provides a male connector comprising: a side hole needle having a hollow needle with an inner hole and an opening through which the inner hole is opened sideways at a tip of the hollow needle, the side hole needle being configured to be inserted into a female connector as a connection partner so as to connect a fluid flow path thereof with that of the female connector; an elastic cover valve body provided so as to be movable in an axial direction with respect to the side hole needle, the elastic cover valve body being configured to cover the opening of the inner hole at a tip of the side hole needle; an urging means for urging the cover valve body from a base end side toward a tip side where the cover valve body covers the opening of the inner hole of the side hole needle; and a limiting member that is superposed on a base end surface of the cover valve body and limits deformation of the base end surface.

According to the male connector of the present preferred embodiment, even if the cover valve body is urged from the base end side, the deformation of the base end surface of the cover valve body is restricted by the limiting member. It is possible to prevent the formation of a gap between the side hole needle and the cover valve body, and prevent the drug solution from leaking or exposing to the proximal end side through the gap. Further, for example, when the urging means is fixed to the cover valve body via the limiting member, it is possible to avoid local deformation of the cover valve body by the urging means, thereby making it difficult to create a gap between the side hole needle and the cover valve body.

An eleventh preferred embodiment provides a male connector comprising: a side hole needle having a hollow needle with an inner hole and an opening through which the inner hole is opened sideways at a tip of the hollow needle, the side hole needle being configured to be inserted into a female connector as a connection partner so as to connect a fluid flow path thereof with that of the female connector; an elastic cover valve body provided so as to be movable in an axial direction with respect to the side hole needle, the elastic cover valve body being configured to cover the opening of the inner hole at a tip of the side hole needle; and an annular sealing portion for sealing between the cover valve body and an outer peripheral surface of the side hole needle, the annular sealing portion being provided on a base end side of the cover valve body.

According to the male connector of the present preferred embodiment, even if the chemical solution leaks from the gap between the cover valve body and the side hole needle, the annular sealing portion can prevent further leakage and exposure of the chemical solution.

A twelfth preferred embodiment provides a male connector comprising: a side hole needle having a hollow needle with an inner hole and a plurality of openings through which the inner hole is opened sideways at a tip of the hollow needle, the side hole needle being configured to be inserted into a female connector as a connection partner so as to connect a fluid flow path thereof with that of the female connector; and an elastic cover valve body provided so as to be movable in an axial direction with respect to the side hole needle, the elastic cover valve body being configured to cover the opening of the inner hole at a tip of the side hole needle, wherein the plurality of openings are formed on a same circumference, and have a flow path cross-sectional area of 70% or less of that of the inner hole of the side hole needle.

According to the male connector of the present preferred embodiment, the cross-sectional area of the plurality of openings of the side hole needle is made smaller than a predetermined size, so that the risk of leakage of the chemical solution from the openings can be reduced. Further, by providing a plurality of such openings on the circumference, a sufficient amount of fluid flowing through the side hole needle can be secured.

A thirteenth preferred embodiment provides a male connector comprising: a side hole needle having a hollow needle with an inner hole and an opening through which the inner hole is opened sideways at a tip of the hollow needle, the side hole needle being configured to be inserted into a female connector as a connection partner so as to connect a fluid flow path thereof with that of the female connector; and an elastic cover valve body provided so as to be movable in an axial direction with respect to the side hole needle, the elastic cover valve body being configured to cover the opening of the inner hole at a tip of the side hole needle, wherein inner and outer diameters of the tip of the side hole needle inserted into the cover valve body are smaller than those of a proximal end portion of the side hole needle.

According to the male connector of the present preferred embodiment, the tip of the side hole needle, which is to be inserted into the cover valve body and the valve member of the female connector, is formed to have a small diameter, whereby the flow efficiency of the fluid in this small diameter portion is lowered, thereby reducing a risk of a leakage of the chemical liquid. Since the base end portion of the side hole needle, which is not to be inserted into the cover valve body and the valve member of the female connector, is formed to have a large diameter, it is possible to obtain a sufficient fluid amount flowing through the side hole needle.

A fourteenth preferred embodiment provides a male connector comprising: a side hole needle having a hollow needle with an inner hole and an opening through which the inner hole is opened sideways at a tip of the hollow needle, the side hole needle being configured to be inserted into a female connector as a connection partner so as to connect a fluid flow path thereof with that of the female connector; and an elastic cover valve body provided so as to be movable in an axial direction with respect to the side hole needle, the elastic cover valve body being configured to cover the opening of the inner hole at a tip of the side hole needle, wherein the cover valve body is formed more flexibly than a valve member of the female connector.

According to the male connector of the present preferred embodiment, when the side hole needle is inserted in a state where the cover valve body and the valve member are overlapped, the cover valve body formed more flexibly than the valve member follows the deformation of the valve member. This makes it possible to reduce a possibility of occurrence of a gap between the cover valve body and the valve member, thereby preventing leakage or exposure of the chemical solution more effectively.

### EFFECT OF THE INVENTION

According to the present invention, it is possible to provide a male connector capable of solving at least one of the problems inherent in a male connector having a conventional structure. For example, the flow path can be stably closed when the female connector is not connected, the stability, reliability, and operability of the connection operation can be improved, the blocking performance with respect to the external space of the flow path when connected to the female connector can be improved, and/or a leakage of a chemical solution can be more reliably prevented.

### BRIEF DESCRIPTION OF THE DRAWINGS

FIG. 1 is a perspective view showing a male connector in the initial state according to a first practical embodiment of the present invention.
FIG. 2 is a perspective view of the male connector shown in FIG. 1 from another direction.
FIG. 3 is a front view of the male connector shown in FIG. 1.
FIG. 4 is a vertical cross-sectional view of the male connector shown in FIG. 1.
FIG. 5 is a vertical cross-sectional view showing a specific example of a female connector that can be connected to the male connector shown in FIG. 1.
FIG. 6 is a vertical cross-sectional view showing a connection state between the male connector shown in FIG. 1 and the female connector shown in FIG. 5.
FIG. 7 is a perspective view showing a male connector in the initial state according to a second practical embodiment of the present invention.
FIG. 8 is a front view of the male connector shown in FIG.7.
FIG. 9 is a left side view of the male connector shown in FIG. 7.
FIG. 10 is a right side view of the male connector shown in FIG. 7.
FIG. 11A is a cross-sectional view taken along the line 9-9 in FIG. 9, and FIG. 11B is a side view showing a single cover valve body.
FIG. 12 is a cross-sectional view taken along the line 12-12 in FIG. 9.
FIG. 13 is a cross-sectional view taken along the line 13-13 in FIG. 8.
FIG. 14 is a perspective view showing a restraining member constituting the male connector shown in FIG. 7.
FIG. 15 is a vertical cross-sectional view showing a specific example of a female connector that can be connected to the male connector shown in FIG. 7.
FIG. 16A is a vertical cross-sectional view showing a connection state between the male connector shown in FIG. 7 and the female connector shown in FIG. 15, and FIG. 16B are X-ray fluoroscopic images of an prototype of the embodiment exemplifying the state before and after the actual connection.
FIG. 17 is a perspective view showing a male connector in the initial state according to a third practical embodiment of the present invention.
FIG. 18 is a vertical cross-sectional view of the male connector shown in FIG. 17.
FIG. 19 is an exploded perspective view of the male connector shown in FIG. 17.
FIG. 20 is a perspective view showing a male connector in the initial state according to a fourth practical embodiment of the present invention.
FIG. 21 is a vertical cross-sectional view showing a state in which a female connector is connected to the male connector shown in FIG. 20.
FIG. 22 is a vertical cross-sectional view showing a state in which a side hole needle of the male connector shown in FIG. 21 is inserted into the female connector.
FIG. 23 is a vertical cross-sectional view which shows another practical embodiment of the male connector of this invention, and is the figure corresponding to FIG. 4.
FIG. 24 is a cross-sectional view taken along the line 24-24 in FIG. 23.
FIG. 25 is a vertical cross-sectional view which shows still another practical embodiment of the male connector of this invention, and is the figure corresponding to FIG. 4.
FIG. 26 is a cross-sectional view taken along the line 26-26 in FIG. 25.
FIG. 27 is a vertical cross-sectional view which shows yet another practical embodiment of the male connector of this invention, and is the figure corresponding to FIG. 4.
FIG. 28 is a vertical cross-sectional view which shows still yet another practical embodiment of the male connector of this invention, and is the figure corresponding to FIG. 4.
FIGS. 29A-29C show a specific example of the cover valve body which can be adopted in the male connector of this invention, wherein FIG. 29A is a front view, FIG.29B is a rear view, and FIG. 29C is a cross-sectional view.

### EMBODIMENTS FOR CARRYING OUT THE INVENTION

Hereinafter, in order to clarify the present invention in more detail, practical embodiments of the present invention will be described in detail with reference to the drawings.

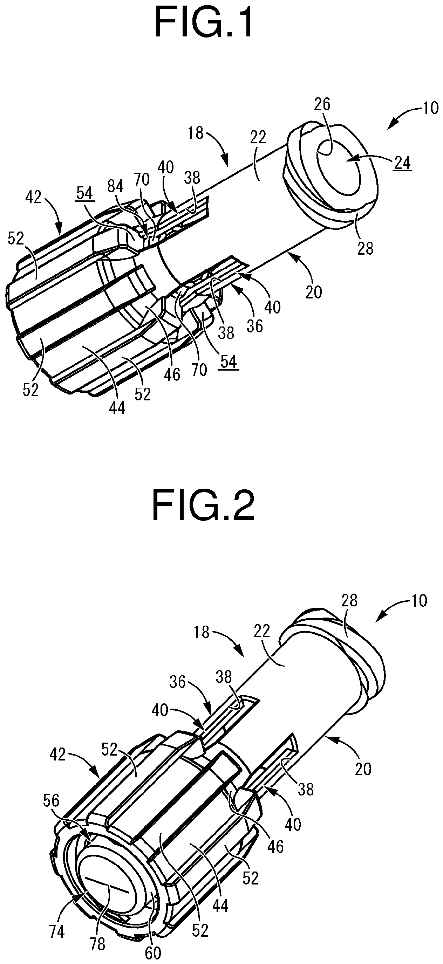

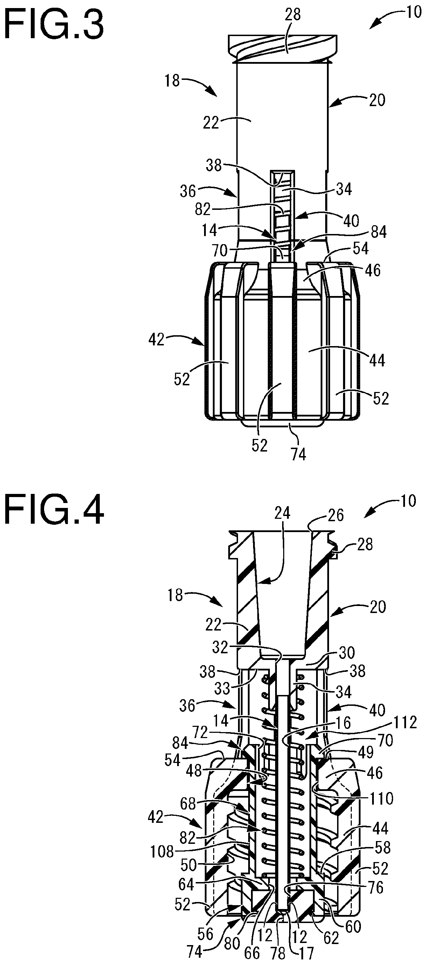

First, FIGS. 1 to 4 show a male connector 10 as a first practical embodiment of the present invention. The male connector 10 includes a side hole needle 14 as a hollow needle having an opening 12 on the side. This side hole needle 14 or a member (details will be described later) within which the side hole needle 14 is inserted is configured to work as a male luer. Namely, by inserting the side hole needle 14 or the member into a female luer of a female connector as a connection partner, the male connector 10 and the female connector are connected to each other so that the fluid flow path is in a communicative state. In the following description, an axial direction means the vertical direction in FIG. 3, which is the central axial direction of the side hole needle 14. Further, a distal end side refers to the lower side in FIG. 3 on which the opening 12 is provided in the side hole needle 14, while a proximal end side refers to the upper side in FIG. 3 on which the side hole needle 14 is fixed.

More specifically, the side hole needle 14 is a hollow needle formed of a metal such as stainless steel or a hard synthetic resin, and has an inner hole 16 extending in its central axis direction. The tip of the side hole needle 14 is closed and rounded so that it can be easily inserted into a valve member 94 of a female connector 86 described later. A tip surface 17 of the side hole needle 14 is formed of a curved surface. The tip portion of the side hole needle 14 is provided with an opening 12 that opens in the direction perpendicular to the axis (sideways), and the inner hole 16 communicates with the external space through the opening 12. In the present practical embodiment, a pair of openings 12, 12 are provided on both sides in one direction perpendicular to the axis (both sides in the left-right direction in FIG. 4).

The male connector 10 has a male connector housing 18 that fixedly supports the side hole needle 14. The male connector housing 18 is a substantially tubular integrally molded product as a whole, which is made of a hard synthetic resin or the like, and a female luer portion 20 having a substantially tubular shape is provided at a base end portion. That is, the female luer portion 20 includes a substantially tubular luer peripheral wall portion 22 extending in the axial direction, and the inner peripheral surface of the luer peripheral wall portion 22 gradually expands toward the base end side, thereby providing a female luer taper surface 24. Further, a base end side opening portion 26 of the female luer portion 20 is provided with a male screw portion 28 protruding from an outer peripheral surface of the luer peripheral wall portion 22 to the outer peripheral side. As a result, a luer lock type syringe, a connector, or the like provided at the end of a flow path extending from a bag or the like in which a chemical solution or the like is sealed can be connected to the female luer portion 20. The connection between the female luer portion 20 and the flow path extending from the bag or the like may be performed before or after the connection between the male connector 10 and the female connector 86, which will be described later.

On the other hand, an annular bottom wall portion 30 projecting to the inner peripheral side is provided at the tip of the luer peripheral wall portion 22, and a through hole 32 penetrating in the axial direction is formed in the center of the bottom wall portion 30. That is, the inner diameter dimension of the luer peripheral wall portion 22 is gradually reduced toward the tip side by the female luer taper surface 24, and is further reduced at the formation position of the bottom wall portion 30, which is the same as the inner diameter dimension of the through hole 32. In the present practical embodiment, the end surface on the tip end side of the bottom wall portion 30 is an annular flat surface 33 extending in the direction perpendicular to the axis.

A needle hub portion 34 that protrudes toward the tip end is provided from the inner peripheral edge portion (opening edge portion of the through hole 32) of the bottom wall portion 30. The inner diameter of the needle hub portion 34 is equal to the inner diameter of the through hole 32, and is substantially equal to or slightly smaller than the outer diameter of the side hole needle 14. As a result, the base end portion of the side hole needle 14 is inserted from the tip end side opening of the needle hub portion 34 in a substantially press-fitted state, and is fixed by adhesion or welding as necessary. Accordingly, the base of the side hole needle 14 is fixedly supported by the needle hub portion 34 and thus the male connector housing 18. Then, as the side hole needle 14 is fixedly supported by the needle hub portion 34 in this way, the inner hole of the female luer portion 20 is held in communication with the inner hole 16 of the side hole needle 14 via the through hole 32 and the inner hole of the needle hub portion 34.

A substantially tubular outer tubular portion 36 is provided in the axially intermediate portion of the male connector housing 18. The outer tubular portion 36 has a substantially tubular shape as a whole, and extends continuously from the distal end side end surface of the bottom wall portion 30 to the distal end side with an outer diameter dimension substantially equal to that of the luer peripheral wall portion 22. As a result, the female luer portion 20 (luer peripheral wall portion 22) and the outer tubular portion 36 are smoothly continuous on the outer peripheral surface. Further, in the axially intermediate portion of the male connector 10 (male connector housing 18), at the forming position of the needle hub portion 34, the needle hub portion 34 and the outer tubular portion 36 are separated from each other in the direction perpendicular to the axis, and are in an internal / external insertion state, thereby producing a double-cylinder structure.

The outer tubular portion 36 is provided with a notch 38 that extends over substantially the entire length in the axial direction and penetrates in the thickness direction. The notch 38 has a substantially rectangular shape that is elongated in the axial direction in which the axial dimension is larger than the width direction dimension. An opening on the base end side of the notch 38 is closed by the bottom wall portion 30 of the female luer portion 20, and a tip end side opening of the notch 38 is closed by an upper bottom wall portion 46 of a lock portion 42, which are described later. As a result, the outer tubular portion 36 (the axially intermediate portion of the male connector housing 18) is formed with substantially rectangular opening windows 40 penetrating in the thickness direction thereof. In the present practical embodiment, opening windows 40 are formed four locations on the periphery of the outer tubular portion 36, at substantially equal intervals (approximately every 90 degrees) in the circumferential direction thereof.

The tip of the outer tubular portion 36 has a tapered tubular shape that slightly expands toward the tip. As a result, even when engaging claws 70 are located at the tip of the opening windows 40 (the tip portion of the outer tubular portion 36) in the initial state of the male connector 10 described later, the engaging claws 70 are more reliably placed within the outer tubular portion 36, thereby preventing disengagement of the engaging claws 70 from the upper bottom wall portion 46 due to contact with an outside member and unintentional drops of a restraining member 56 from the male connector housing 18.

Furthermore, the lock portion 42 is formed continuously from and integrally with the tip of the outer tubular portion 36 at the tip portion of the male connector housing 18. The lock portion 42 has a substantially tubular shape extending in the axial direction as a whole. In the present practical embodiment, the lock portion 42 has a tubular shape having a diameter larger than that of the female luer portion 20. That is, the lock portion 42 includes a substantially tubular lock peripheral wall portion 44 extending in the axial direction, and the opening on the base end side of the lock peripheral wall portion 44 has the annular upper bottom wall portion 46 protruding inward. A through hole 48 penetrating in the axial direction is formed in the center of the upper bottom wall portion 46. The inner diameter dimension of the through hole 48 is larger than the outer diameter dimension of the side hole needle 14, and is smaller than the inner diameter of the outer tubular portion 36. In the present practical embodiment, the base end side end surface of the upper bottom wall portion 46 is an annular flat surface 49 extending in the direction perpendicular to the axis.

An female screw portion 50 is provided on the inner peripheral surface of the lock peripheral wall portion 44. The outer peripheral surface of the lock peripheral wall portion 44 is provided with ridges 52 protruding toward the outer peripheral side with a substantially rectangular cross section over a substantially entire axial length of the lock peripheral wall portion 44. In the present practical embodiment, the eight ridges 52 are formed at substantially equal intervals (approximately every 45 degrees) in the circumferential direction, whereby the outer peripheral surface of the lock peripheral wall portion 44 is provided with irregularities. By providing irregularities or unevenness on the outer peripheral surface of the lock portion 42 (lock peripheral wall portion 44) in this way, it is easy to perform an operation of grasping the male connector 10 and screwing it into the female connector 86, described later.

In particular, in the present practical embodiment, the base end side end surface of the ridge 52 is a flat surface 54 extending in the direction perpendicular to the axis, and the flat surface 54 is smoothly continuous with the flat surface 49 which is the base end side end surface of the upper bottom wall portion 46 in the direction perpendicular to the axis.

In the state where the side hole needle 14 is fixed to the needle hub portion 34 of the male connector housing 18, in the present practical embodiment, the tip surface 17 of the side hole needle 14 is at a position substantially equal to the tip of the lock portion 42 in the axial direction, or at a position slightly located on the base end side. However, the tip surface 17 of the side hole needle 14 may protrude to the tip side from the lock portion 42.

The restraining member 56 is assembled to the male connector housing 18 having such a structure. The restraining member 56 is, for example, a substantially tubular member made of a hard synthetic resin, and extends substantially straight in the axial direction. An annular stepped surface 58 extending in the direction perpendicular to the axis is formed on the outer peripheral surface of the tip end portion of the restraining member 56, and the tip side of the stepped surface 58 has a larger outer diameter than the proximal end side of the stepped surface 58. A portion of the tip portion of the restraining member 56 having an increased outer diameter is defined as an outer peripheral wall portion 60 that extends continuously over the entire circumference in the circumferential direction. The axial dimension of the outer peripheral wall portion 60 is not limited in any way, but it is preferably larger than the diameter of the opening 12 of the side hole needle 14.

At the tip of the outer peripheral wall portion 60, an annular locking claw 62 protruding toward the inner peripheral side is formed. At the base end portion of the outer peripheral wall portion 60, on the other hand, there is formed an annular bottom wall portion 64 protruding toward the inner peripheral side. That is, a through hole 66 is formed at the inner peripheral side (center) of the bottom wall portion 64 penetrating in the axial direction. The inner diameter of the through hole 66 is larger than the outer diameter of the side hole needle 14, and is smaller than the inner diameter of the coil spring 82, which will be described later. The portion of the restraining member 56 on the proximal end side of the stepped surface 58 is a proximal end side protrusion 68 extending from the outer peripheral wall portion 60 and the bottom wall portion 64 toward the proximal end side. By providing the bottom wall portion 64, it is possible to improve the function of the restraining member 56, such as stabilizing the deformation mode of the cover valve body 74, which will be described later. Further, it prevents an application of external force from the coil spring 82 to the base end of the cover valve body 74, and reduces the risk of exposure due to deformation of the cover valve body 74.

The proximal end side protrusion 68 has a substantially tubular shape extending in the axial direction as a whole. As described above, the proximal end side protrusion 68 has an outer diameter dimension smaller than the outer diameter dimension of the outer peripheral wall portion 60, and has an inner diameter also smaller than the inner diameter dimension of the outer peripheral wall portion 60. In the present practical embodiment, the outer diameter dimension of the proximal end side protrusion 68 is substantially equal to or slightly smaller than the inner diameter dimension of the through hole 48 provided in the center of the upper bottom wall portion 46 of the lock portion 42. The inner diameter of the proximal end side protrusion 68 is larger than the outer diameter of the coil spring 82, which will be described later.

Further, the engaging claws 70 projecting to the outer peripheral side are provided at the base end of the proximal end side protrusion 68. The engaging claws 70 are partially formed on the circumference, and in the present practical embodiment, the four engaging claws 70 are formed at substantially equal intervals (approximately every 90 degrees) in the circumferential direction. On the other hand, between the engaging claws 70, 70 adjacent to each other in the circumferential direction, there are formed notches 72 extending from the base end of the proximal end side protrusion 68 toward the tip end side, with a given width dimension and a give axial dimension. As a result, the portion where engaging claws 70 are formed at the base end portion of the proximal end side protrusion 68 can be elastically deformed in the direction perpendicular to the axis, whereby, as will be described later, the assembly of the restraining member 56 to the male connector housing 18 can be readily performed.

A cover valve body 74 is assembled in a region surrounded by the outer peripheral wall portion 60 and the bottom wall portion 64 in the tip portion of the restraining member 56 having such a structure. The cover valve body 74 has a substantially disk shape as a whole, and is formed of an elastic body such as rubber or an elastomer. An insertion recess 76 extending toward the tip end is formed in the central portion of the end face of the cover valve body 74 on the base end side. The insertion recess 76 has a shape corresponding to the tip portion of the side hole needle 14, extends linearly from the opening, and has a curved surface on the bottom surface. The insertion recess 76 does not penetrate the cover valve body 74, and the bottom surface of the insertion recess 76 is located at an axially intermediate portion of the cover valve body 74. On the other hand, a slit 78 extending in the axial direction and penetrating the cover valve body 74 is formed from the top (deepest portion) of the bottom surface of the insertion recess 76. That is, it is also possible to grasp that the insertion recess 76 and the slit 78 form a slit that penetrates the entire cover valve body 74 in the axial direction.

In the present practical embodiment, as shown in FIG. 2, in the central portion of the cover valve body 74, the slit 78 extends in a straight line shape in the direction perpendicular to the axis, but the shape of the slit is not limited in any way. Instead, it may extend in a cross shape or radially from the center in a plurality of directions. However, such a slit 78 is not essential. Further, the insertion recess 76 is not essential.

Further, a locking recess 80 that opens to the outer peripheral side is formed on the outer peripheral surface of the locking recess cover valve body 74. The locking recess 80 has a shape corresponding to the locking claw 62 provided at the tip of the restraining member 56. In the present practical embodiment, the locking recess 80 is continuously formed in an annular shape over the entire circumference in the circumferential direction. However, such a locking recess does not need to be formed at the time of molding the cover valve body. Namely, the locking recess may be formed by pressing the locking claw against the outer peripheral surface of the flat cover valve body so that the locking claw is formed on the outer peripheral surface of the cover valve body by biting of the locking claw against the cover valve body. A strong compressive force is generated in an annular shape on the tip end side of the outer peripheral surface of the cover valve body 74 by the locking claw 62, reducing the risk of exposure from the tip end side of the cover valve body 74.

The cover valve body 74 as described above is assembled to the restraining member 56. That is, the cover valve body 74 having a substantially disk shape is fitted in the region of the restraining member 56 surrounded by the outer peripheral wall portion 60 and the bottom wall portion 64. The outer peripheral surface of the cover valve body 74 is abutted on the outer peripheral wall portion 60, the outer peripheral side of the cover valve body 74 is covered by the outer peripheral wall portion 60 over the entire circumference in the circumferential direction, and the bottom surface (base end side end surface) of the cover valve body 74 is abutting on the bottom wall portion 64, and the bottom surface side (base end side) of the cover valve body 74 is covered with the bottom wall portion 64. Further, by inserting the locking claw 62 of the restraining member 56 into the locking recess 80 of the cover valve body 74, the cover valve body 74 is prevented from being unintentionally dropped from the restraining member 56. As a result, the restraining member 56 and the cover valve body 74 move integrally in the axial direction when the male connector 10 and the female connector 86, which will be described later, are screwed and unscrewed. By assembling the cover valve body 74 to the restraining member 56 as described above, it is preferable that the cover valve body 74 is compressed in the direction perpendicular to the axis by the outer peripheral wall portion 60, or the cover valve body 74 is compressed by the bottom wall portion 64 and the locking claw 62 in the axial direction, but, the cover valve body 74 does not have to be compressed in the direction perpendicular to the axis and/or in the axial direction between these members, and may be held between these members in a state of a so-called zero touch or alternatively with a gap. In the present practical embodiment, the cover valve body 74 projects to the tip end side of the locking claw 62 and covers the tip end side end surface of the outer peripheral wall portion 60.

Then, the assembling body of the restraining member 56 and the cover valve body 74 is assembled to the male connector housing 18 to which the side hole needle 14 is fixed. That is, the proximal end side protrusion 68 of the restraining member 56 is inserted into the through hole 48 of the lock portion 42 in the male connector housing 18. As a result, the engaging claw 70 protruding from the base end of the proximal end side protrusion 68 to the outer peripheral side is brought into contact with the inner peripheral surface of the through hole 48, and the formed portions of the engaging claws 70 are elastically deformed to the inner peripheral side. As a result, the proximal end side protrusion 68 can pass through the through hole 48, and when the engaging claws 70 pass through the through hole 48, the engaging claws 70 are elastically restored and deformed, so that the engaging claws 70 enter the opening windows 40 that open in the middle portion in the axial direction of the male connector housing 18. This prevents the assembly of the restraining member 56 and the cover valve body 74 from falling off from the male connector housing 18. The outer diameter dimension at the formation positions of the engaging claws 70 (assumed outer diameter dimension assuming an annular surface connecting the outer peripheral surfaces of each engaging claws 70) is larger than the inner diameter dimension of the outer tubular portion 36. As a result, when the male connector 10 and the female connector 86 are screwed together, which will be described later, the engaging claws 70 come into contact with the inner peripheral surface of the opening windows 40, so that the male connector housing 18 and the restraining member 56 rotate integrally.

Further, the side hole needle 14 extending toward the tip end side from the needle hub portion 34 of the male connector housing 18 is inserted through the restraining member 56 and the cover valve body 74.

Here, a coil spring 82 as an urging means is arranged between the male connector housing 18 and the restraining member 56 in the axial direction. The coil spring 82 is externally placed about the side hole needle 14, and specifically, the base end portion of the coil spring 82 is externally placed about the needle hub portion 34 of the female luer portion 20, and fixed to the flat surface 33 which is the end surface on the tip end side of the bottom wall portion 30. The tip of the coil spring 82 is fixed to the base end side end surface of the bottom wall portion 64 of the restraining member 56, for example. In the initial state shown in FIGS. 1 to 4, the coil spring 82 is in a substantially natural length or in a compressed state, and the restraining member 56 and the cover valve body 74 are urged by the coil spring 82 in the direction of displacement toward the tip side with respect to the male connector housing 18.

In such an initial state, the restraining member 56 is urged toward the tip end side with respect to the male connector housing 18, and the engaging claw 70 at the proximal end side protrusion 68 is brought into abutting contact with the flat surface 49 as the base end side end surface in the upper bottom wall portion 46 of the lock portion 42, in order to restrict movement of the restraining member 56 toward the tip end. That is, in the present practical embodiment, a restricting means 84 that restricts the movement of the restraining member 56 and the cover valve body 74 toward the tip end includes the engaging claw 70 at the proximal end side protrusion 68.

Further, in such an initial state, the cover valve body 74 is located at the tip of the side hole needle 14. Specifically, the tip of the side hole needle 14 is inserted into the insertion recess 76 of the cover valve body 74, and the tip surface 17 of the side hole needle 14 comes into contact with the bottom surface of the insertion recess 76 in a state of a so-called zero touch or is slightly separated therefrom. As a result, in the initial state, the tip surface 17 of the side hole needle 14 is located at the intermediate portion in the axial direction (direction of penetration of the slit 78) of the cover valve body 74, and the tip surface 17 of the side hole needle 14 is covered with the cover valve body 74, while the slit 78 of the cover valve body 74 is in a closed state. Further, in such a state, the inner peripheral surface of the insertion recess 76 of the cover valve body 74 is brought into contact with the opening 12 of the side hole needle 14 in a substantially close contact state, and the opening of the side hole needle 14 to the outside is closed. Further, in the present practical embodiment, in the initial state, the outer peripheral wall portion 60 of the restraining member 56 is located at such an axial position as to cover the opening 12 of the side hole needle 14, but the present practical embodiment is not limited to this mode. Furthermore, in the present practical embodiment, in the initial state, the end face on the tip end side of the cover valve body 74 is located on the tip end side of the lock portion 42.

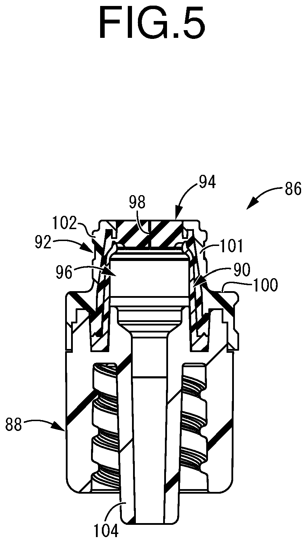

The male connector 10 of the present practical embodiment having the structure as described above in the initial state is connected to the female connector, which is the connection partner, so that the fluid flow path is in a communicating state. The female connector that can be connected to the male connector 10 of the present practical embodiment is not limited in any way, but a specific example of the female connector is shown in FIG. 5.

The female connector 86 of the present practical embodiment includes a base member 88, a lower cover member 90, an upper cover member 92, and the valve member 94. That is, the upper and lower cover members 92, 90 are fixed to the base member 88 with the valve member 94 sandwiched between them, and the valve member 94 is arranged in the opening of a female luer portion 96 constituted by the upper and lower cover members 92, 90. The valve member 94 is penetrated by a slit 98 in the thickness direction, and the slit 98 is closed in the initial state. A stepped surface 100 is provided on the outer peripheral surface of the upper cover member 92 of the present practical embodiment, and the base end side of the stepped surface 100 (upper side in FIG. 5) is a small-diameter tubular portion 101. On the outer peripheral surface of the small-diameter tubular portion 101, a male screw portion 102, which is configured to be screwed with the female screw portion 50 provided in the lock portion 42 of the male connector 10, is provided. Further, the base member 88 is provided with a tubular portion 104 to which a catheter (not shown) or the like is connected, and the catheter is connected to a blood vessel or the like of a patient.

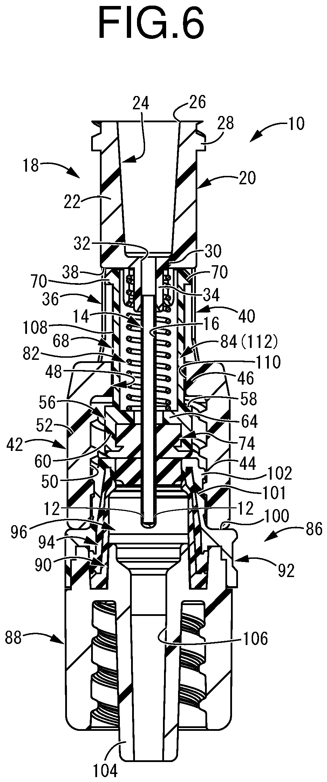

By connecting the male connector 10 to the female connector 86 as described above, a fluid flow path 106 is in a communicating state as shown in FIG. 6. That is, from the state in which the tip end surface of the male connector 10 and the base end surface of the female connector 86 (upper end surface in FIG. 5) are overlapped with each other, the male connector 10 and the female connector 86 are pressed against each other in the direction of approach, and by rotating the male connector 10 with respect to the female connector 86, the male screw portion 102 of the female connector 86 is screwed into the female screw portion 50 of the lock portion 42 of the male connector 10. As a result, the small-diameter tubular portion 101 of the female connector 86 is screwed into the lock portion 42. The screwing engagement between the male screw portion 102 and the female screw portion 50 is restricted by, for example, the end surface on the tip end side of the lock portion 42 coming into contact with the stepped surface 100 provided on the outer peripheral surface of the female connector 86 (upper cover member 92).

Then, as described above, the small-diameter tubular portion 101 of the female connector 86 is inserted into the lock portion 42, so that the end faces on the base end side of the upper cover member 92 and the valve member 94, which are the end faces on the base end side of the female connector 86, come into contact with the tip end surface of the cover valve body 74 inside the lock portion 42, and further, the restraining member 56 and the cover valve body 74 are pushed toward the base end side against the urging force of the coil spring 82 (while the coil spring 82 is being compressed). Specifically, the base end side end face of the upper cover member 92, which is a rigid member, abuts on the tip end side end face of the restraining member 56, which is also a rigid member, via the cover valve body 74, so as to push the restraining member 56 and the cover valve body 74 toward the base end side. However, in the present practical embodiment, in the initial state, the tip end surface of the cover valve body 74 is located on the tip side of the lock portion 42. Thus, after the tip end face of the cover valve body 74 and the base end faces of the upper cover member 92 and the valve member 94 come into contact, the small-diameter tubular portion 101 of the female connector 86 is inserted into the lock portion 42, and the restraining member 56 and the cover valve body 74 are pushed toward the base end side. As a result, the cover valve body 74 and the valve member 94 are in close contact with each other in a liquid-tight manner thereby preventing the drug solution such as an anticancer drug from being exposed or leaking through the gap between the cover valve body 74 and the valve member 94.

As a result, the restraining member 56 and the cover valve body 74 are moved toward the proximal end side with respect to the side hole needle 14, and the tip of the side hole needle 14 protrudes toward the tip end side of the cover valve body 74 through the slit 78, while the tip of the side hole needle 14 is inserted through the valve member 94 via the slit 98 and inserted into the female luer portion 96 of the female connector 86. Then, the tip of the side hole needle 14, which has been covered with the cover valve body 74, becomes exposed inside the female luer portion 96, so that the openings 12, 12 of the side hole needle 14 are opened in the female luer portion 96. As a result, the internal space of the female luer portion 20 in the male connector 10 is communicated with the internal space of the female luer portion 96 in the female connector 86 through the inner hole 16 of the side hole needle 14. As a result, the fluid flow path 106 including the female luer portion 20 of the male connector 10, the side hole needle 14, the female luer portion 96 of the female connector 86, the tubular portion 104, and the like is brought into a communicating state, and a drug solution such as an anticancer drug enclosed in a bag connected to the female luer portion 20 of the male connector 10 is injected into the blood vessel of the patient through a catheter or the like connected to the fluid flow path 106 and the tubular portion 104.

The movement of the restraining member 56 and the cover valve body 74 toward the proximal end side with respect to the side hole needle 14 may be restricted by, for example, the engaging claw 70 provided at the proximal end of the restraining member 56 abutting on the flat surface 33 of the bottom wall portion 30 of the female luer portion 20, or by the stepped surface 58 provided on the outer peripheral surface of the restraining member 56 abutting on the end face on the tip end side of the upper bottom wall portion 46 of the lock portion 42. Further, the outer diameter dimension of the proximal end side protrusion 68 of the restraining member 56 is substantially equal to or slightly smaller than the inner diameter dimension of the through hole 48 of the lock portion 42. Thus, the movement of the restraining member 56 and the cover valve body 74 toward the proximal end side is performed substantially linearly in the axial direction while an outer peripheral surface 108 of the proximal end side protrusion 68 of the restraining member 56 is substantially abutting on an inner peripheral surface 110 of the through hole 48. That is, in the present practical embodiment, a guide portion that guides the movement of the restraining member 56 toward the proximal end side includes the outer peripheral surface 108 of the proximal end side protrusion 68.

Then, after injecting the drug solution into the patient as described above, the fluid flow path 106 is shut off by disconnecting the male connector 10 and the female connector 86. That is, by unscrewing the female screw portion 50 of the lock portion 42 of the male connector 10 and the male screw portion 102 of the female connector 86, the small-diameter tubular portion 101 of the female connector 86 is moved to the outside of the lock portion 42, thereby releasing the contact between the tip end surface of the male connector 10 and the proximal end surface of the female connector 86. As a result, the restraining member 56 and the cover valve body 74 are moved toward the tip end side with respect to the side hole needle 14 according to the urging force of the coil spring 82, and the movement of the restraining member 56 toward the tip end side is restricted by the restricting means 84. By so doing, the cover valve body 74 is returned to the initial position covering the tip surface 17 of the side hole needle 14. That is, in the present practical embodiment, a positioning means 112 that allows the cover valve body 74 to move to the proximal end side of the side hole needle 14 and elastically positions the cover valve body 74 to the tip of the side hole needle 14 includes the coil spring 82 serving as the urging means and the restricting means 84. Then, by returning the cover valve body 74 to the initial position in this way, the openings 12, 12 of the side hole needle 14 are covered and closed by the inner peripheral surface of the insertion recess 76 of the cover valve body 74.

In the male connector 10 of the present practical embodiment having the above structure, when the male connector 10 is not connected to the female connector 86, the openings 12, 12 of the side hole needle 14 are covered with the cover valve body 74 made of an elastic body so as to be stably closed. In particular, the outer peripheral side of the cover valve body 74 is covered by the outer peripheral wall portion 60 over the entire circumference, and is supported by being sandwiched between the bottom wall portion 64 and the locking claw 62 in the axial direction. Thus, unintended deformation of the cover valve body 74 is suppressed, making it possible to prevent the openings 12, 12 of the side hole needle 14 from being unintentionally opened to expose or leak a fluid such as a drug solution. Further, when the male connector 10 is not connected to the female connector 86, the tip surface 17 of the side hole needle 14 is covered with the cover valve body 74, thereby preventing scatter of a fluid such as a drug solution adhering to the tip surface 17 by being inserted into the female connector 86.

Further, in the present practical embodiment, the engaging claw 70 is provided at the proximal end of the restraining member 56, and the restricting means 84 is constituted by including the engaging claw 70 so as to restrict movement of the restraining member 56 toward the tip end, while the positioning means 112 is constituted by including the restricting means 84 and the coil spring 82 that is the urging means so as to elastically position the cover valve body 74 at the tip of the side hole needle 14. Therefore, when the male connector 10 and the female connector 86 are connected, the restraining member 56 and the cover valve body 74 are moved toward the proximal end side with respect to the side hole needle 14 to open the openings 12, 12 of the side hole needle 14. Meanwhile, when the male connector 10 and the female connector 86 are disconnected, the restraining member 56 and the cover valve body 74 are automatically moved toward the tip side with respect to the side hole needle 14 to close the openings 12, 12, thereby further reducing the risk of exposure or leakage of a fluid such as a drug solution.

Furthermore, in the present practical embodiment, the guide portion for guiding the axial movement of the restraining member 56 is constituted by including the outer peripheral surface 108 of the proximal end side protrusion 68 of the restraining member 56. Thus, opening and closing of the openings 12, 12 of the side hole needle 14 due to the movement of the cover valve body 74 and the restraining member 56 can be realized more stably.

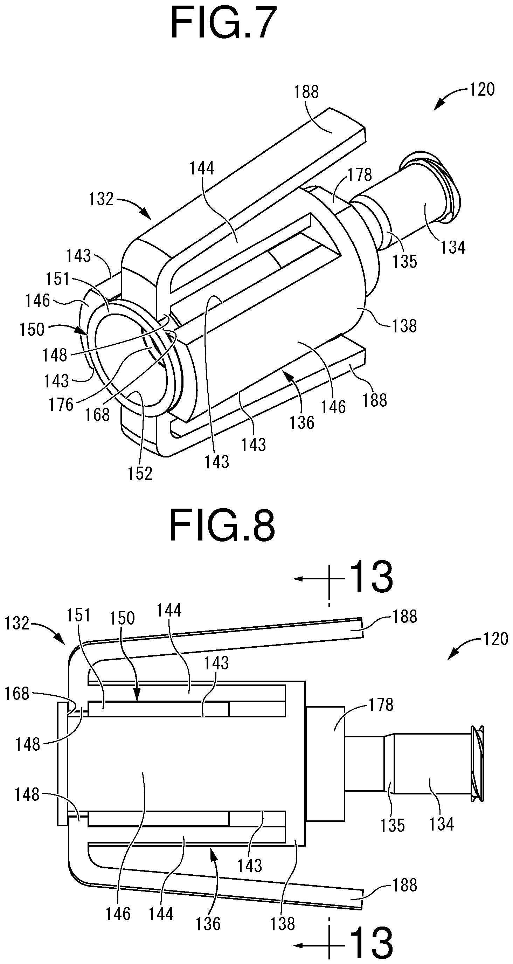

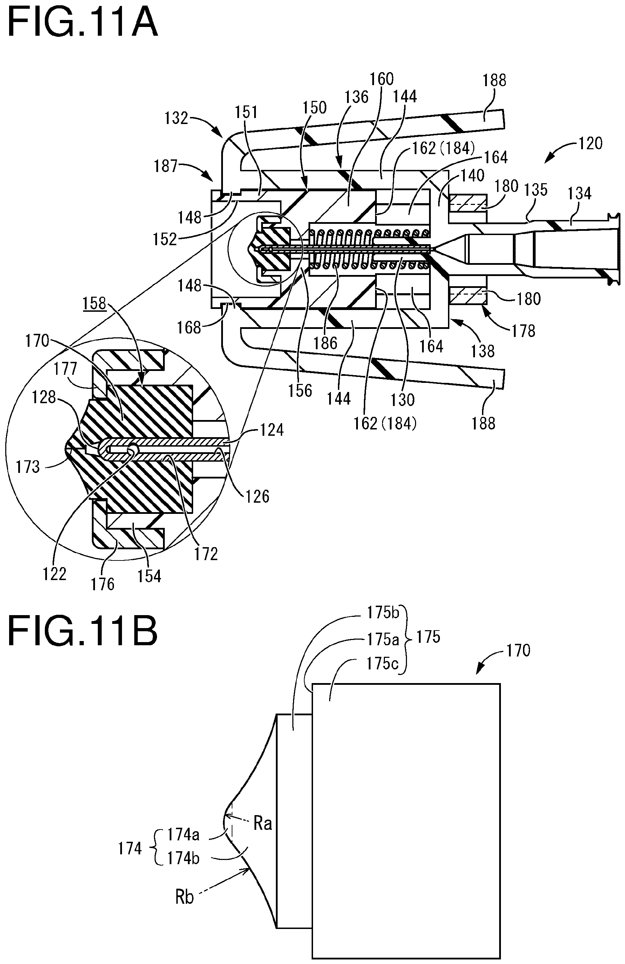

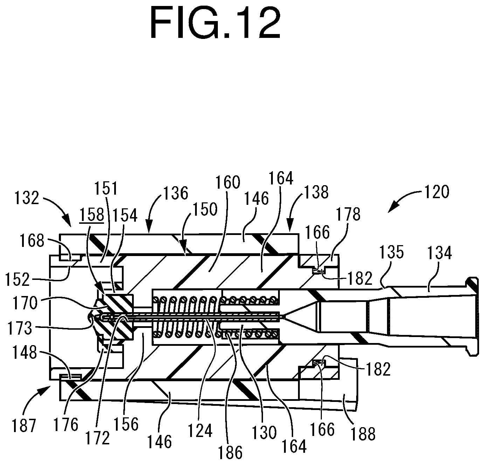

Next, FIGS. 7 to 13 show a male connector 120 as a second practical embodiment of the present invention. The male connector 120 includes a side hole needle 124 as a hollow needle having an opening 122 on the lateral side. In the present practical embodiment, the axial direction means the left-right direction in FIG. 11, which is the central axial direction of the side hole needle 124. Further, the distal end side or tip side refers to the left side in FIG. 11 on which the opening 122 is provided in the side hole needle 124, while the proximal end side or base end side refers to the right side in FIG. 11 on which the side hole needle 124 is fixed.

The side hole needle 124 is a hollow needle formed of a metal such as stainless steel or a hard synthetic resin, and is a tubular body having an inner hole 126 extending in the central axis direction. The tip of the side hole needle 124 is closed and rounded so that it can be easily inserted into a disc valve 198 as a valve member of a female connector 190 described later, and a tip surface 128 of the side hole needle 124 is formed by a curved surface.

The opening 122 that opens in the direction perpendicular to the axis (sideways) is provided at the tip of the side hole needle 124, and the inner hole 126 communicates with the external space through the opening 122. In the present practical embodiment, a pair of openings 122, 122 are provided on both sides in one direction perpendicular to the axis (both sides in the direction orthogonal to the paper surface in FIG. 11).

Further, the male connector 120 includes a needle hub portion 130 that fixedly supports the side hole needle 124, and has a male connector housing 132 that is fixedly provided to the side hole needle 124. The male connector housing 132 is a substantially cylindrical member made of a hard synthetic resin or the like as a whole, and a female luer portion 134 having a substantially cylindrical shape is provided at the base end portion thereof. A luer lock type or luer slip type syringe or connector as an external flow path provided at the end of a flow path extending from a bag or the like in which a drug solution or the like is sealed can be connected to the female luer portion 134.

The needle hub portion 130 of substantially cylindrical shape projecting toward the tip side is provided substantially coaxially at the tip of the female luer portion 134. The proximal end portion of the side hole needle 124 is inserted into the needle hub portion 130 in a substantially press-fitted state, and by being adhered or welded as needed, the side hole needle 124 is fixedly supported by the needle hub portion 130 and thus the male connector housing 132. As a result, the female luer portion 134 and the inner hole 126 of the side hole needle 124 communicate with each other, and the external flow path is connected to the side hole needle 124 via the female luer portion 134.

In the present practical embodiment, the male connector housing 132 is formed as an integrally molded product including the needle hub portion 130 and the female luer portion 134.

Further, in the present practical embodiment, the inner diameter dimension of the female luer portion 134 is reduced toward the tip end side, and at the extreme tip thereof, the inner diameter dimension is substantially equal to that of the side hole needle 124. Furthermore, a tapered surface 135 is formed on the outer peripheral surface of the axially intermediate portion of the female luer portion 134, the outer diameter dimension of the tapered surface 135 gradually decreasing toward the tip end side.

The male connector housing 132 integrally includes an outer tubular portion 136 that is outwardly separated from and arranged substantially coaxially with the side hole needle 124. The outer tubular portion 136 has a substantially bottomed cylinder shape that opens toward the tip side as a whole, and a substantially circular bottom plate portion 138 that extends in the direction perpendicular to the axis is provided at the base end of the outer tubular portion 136.

In the present practical embodiment, the outer peripheral portion of the bottom plate portion 138 is substantially annular and the inner peripheral portion thereof is substantially cross-shaped, so as to be provided with a pair of proximal end wall portions 140, 140 that are mutually orthogonal in two directions perpendicular to the axis (vertical direction and direction orthogonal to the paper surface in FIG. 11). By these proximal end wall portions 140, 140 being orthogonal to each other, there are provided four substantially fan-shaped insertion holes 142 each having a peripheral length around the central axis smaller than 1/4 circumference and are formed at substantially equal intervals in the circumferential direction in the bottom plate portion 138. Further, from the intersection of the proximal end wall portions 140, 140, which is the central portion of the bottom plate portion 138, the female luer portion 134 projects toward the base end side, while the needle hub portion 130 projects toward the tip end side.

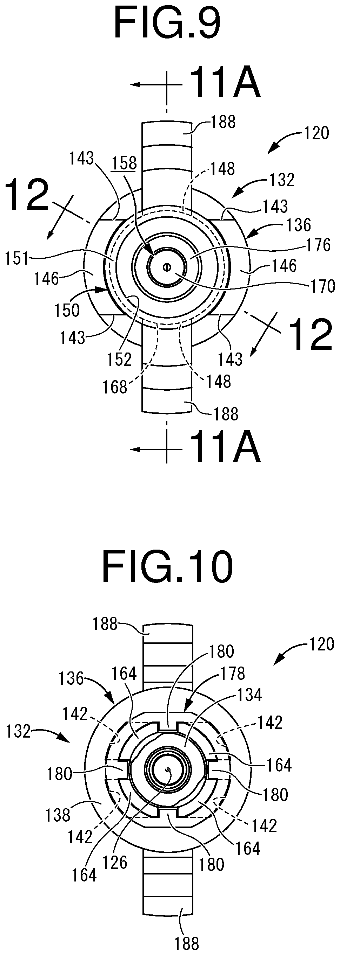

The outer tubular portion 136 is provided with notches 143 that open toward the tip side and penetrate in the thickness direction. In the present practical embodiment, in the outer tubular portion 136, four notches 143 arranged in the circumferential direction are provided over a substantially overall length in the axial direction from the tip end to the base end portion. With these notches 143, the circumferential dimension between the notches 143, 143 that are adjacent to each other in the vertical direction (vertical direction in FIG. 9) of the male connector housing 132 is larger than the circumferential dimension between the notches 143, 143 that are adjacent to each other in the width direction (left-right direction in FIG. 9). The peripheral wall forming the outer tubular portion 136 is divided into four by these notches 143.

That is, a pair of flexible wall portions 144, 144 whose circumferential dimensions are reduced and easily undergoing elastic deformation in the radial direction are provided on both sides of the outer tubular portion 136 in the vertical direction, while a pair of wall portions 146, 146 whose circumferential dimensions are larger than those of the flexible wall portions 144 and having a larger deformation strength in the radial direction are provided on both sides of the outer tubular portion 136 in the width direction. At the tips of the flexible wall portions 144, 144, fitting convex portions 148, 148 are formed as connection engaging portions protruding toward the inner peripheral side.

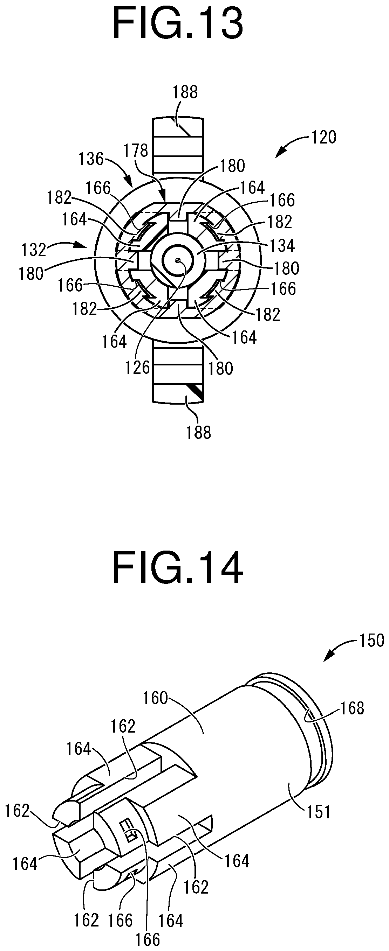

Arestraining member 150 that is axially movable with respect to the side hole needle 124 and the outer tubular portion 136 is provided between the side hole needle 124 and the outer tubular portion 136 in the radial direction. As shown in FIG. 14, the restraining member 150 of the present practical embodiment has a substantially cylindrical shape as a whole, and is externally placed about the side hole needle 124. The outer diameter dimension of the restraining member 150 is substantially equal to or slightly smaller than the inner diameter dimension of the male connector housing 132, and is substantially constant over the substantially overall length in the axial direction.

A tubular tip protruding portion 151 having a relatively large inner diameter dimension is formed in the tip portion of the restraining member 150, and a substantially annular large-diameter recess 152 that opens toward the tip side is formed by the inner peripheral portion of the tip protruding portion 151. This makes it difficult for the user's finger to touch a cover valve body 170, which will be described later. The tip diameter of the tip protruding portion 151 is preferably small so that a finger cannot be easily inserted. The inner diameter of the large-diameter recess 152 is larger than the outer diameter of a male screw portion 208 on the outer peripheral surface of a small-diameter tubular portion 206 of a female connector 190, which will be described later. The inner peripheral edge of the large-diameter recess 152 is provided with a tubular portion 154 projecting toward the tip end, and the base end of the tubular portion 154 is provided with a substantially annular inner peripheral protrusion 156 projecting toward the inner peripheral side. As a result, the inner diameter dimension of the restraining member 150 is reduced in a stepwise manner from the large-diameter recess 152 to the tubular portion 154 and then to the inner peripheral protrusion 156, and the inner diameter dimension of the inner peripheral protrusion 156 is larger than the outer diameter of the side hole needle 124.

The substantially columnar space surrounded by the tubular portion 154 and the inner peripheral protrusion 156 is defined as an accommodating area 158 in which the cover valve body 170 described later is accommodated. Therefore, the cover valve body 170 housed in the accommodating area 158 can be moved axially with respect to the side hole needle 124 together with the restraining member 150.

Further, in the restraining member 150, a proximal end side protrusion 160 having a substantially cylindrical shape is formed on the proximal end side of the inner peripheral protrusion 156. The proximal end side protrusion 160 is formed substantially coaxially with the inner hole of the inner peripheral protrusion 156 and the large-diameter recess 152, and the proximal end side protrusion 160 and the large-diameter recess 152 communicate with each other through the inner hole of the inner peripheral protrusion 156. The inner diameter dimension of the proximal end side protrusion 160 is larger than the inner diameter dimension of the inner peripheral protrusion 156, and is substantially equal to or slightly smaller than the outer diameter dimension of the tip portion of the female luer portion 134.

The restraining member 150 is provided with notches 162 that penetrate the peripheral wall (proximal end side protrusion 160) in the thickness direction so as to extend from the proximal end toward the tip end side. In the present practical embodiment, each notch 162 has an axial dimension shorter than that of the proximal end side protrusion 160, and the inside and outside of the restraining member 150 communicate with each other in the radial direction through each notch 162.

Further, in the present practical embodiment, the notches 162 are provided at the circumferential positions corresponding to the proximal end wall portions 140 of the outer tubular portion 136 of the male connector housing 132, and are formed at substantially equal intervals at four locations in the circumferential direction. As a result, at the proximal end portion of the restraining member 150, the annular peripheral wall (proximal end side protrusion 160) is substantially divided into four, so as to form four leg portions 164 that are separated from one another in the circumferential direction. The cross-sectional shape of each leg portion 164 has a shape substantially corresponding to the insertion hole 142 provided in the bottom plate portion 138 of the outer tubular portion 136, and has a fan shape whose peripheral length around the central axis is smaller than 1/4 circumference.

Further, the outer diameter dimension (outer diameter dimension of the virtual annular surface connecting the outer peripheral surfaces of the leg portions 164) is reduced at the proximal end portion of the leg portions 164, and at the portion where the outer diameter dimension is reduced, positioning recesses 166 extending in the circumferential direction are provided on the respective outer peripheral surfaces.

Further, an annular fitting concave portion 168 extending over the entire circumference in the circumferential direction is formed on the outer peripheral surface of the tip portion (tip protruding portion 151) of the restraining member 150. It should be noted that the fitting concave portion does not have to be provided over the entire circumference in the circumferential direction, and may only be provided on both sides in the vertical direction corresponding to the fitting convex portions 148, 148 of the male connector housing 132.

The cover valve body 170 made of an elastic body such as rubber or an elastomer is arranged in the accommodating area 158 of the restraining member 150 having the above structure. That is, a holding member that holds the cover valve body 170 is constituted by the restraining member 150. The cover valve body 170 has a solid cylindrical shape or a circular plate shape (disk shape) as a whole, and includes an insertion recess 172 extending from substantially the center of the end face on the base end side to the tip end side. The insertion recess 172 has a circular hole shape extending substantially corresponding to the tip of the side hole needle 124 with an inner diameter smaller than the outer diameter of the side hole needle 124, and extends linearly in the axial direction from the opening on the base end side. Further, the tip side of the insertion recess 172 is tapered to reduce the diameter, and the tip (bottom surface) of the insertion recess 172 is located at the axially intermediate portion of the cover valve body 170 without penetrating the cover valve body 170.

Further, in the cover valve body 170, a slit 173 extends from the bottom surface (deepest portion) of the insertion recess 172 so as to penetrate to the tip side in the axial direction. That is, it can be grasped that the insertion recess 172 and the slit 173 form a slit that penetrates the entire cover valve body 170 in the axial direction. The slit 173 is not specifically limited to a straight-line slit, a radial slit, a pin-hole slit, or the like, but is held in a closed state by the elasticity of the cover valve body 170.

Then, the tip of the side hole needle 124 is inserted and accommodated in the insertion recess 172, and the inner peripheral surface of the insertion recess 172 of the cover valve body 170 is elastically in close contact with the outer peripheral surface of the side hole needle 124, so as to seal the opening 122.
The insertion recess 172 is formed deeper than the tip of the side hole needle 124, and a gap is set at the bottom of the insertion recess 172 to cope with fluctuations in the tip position of the side hole needle caused by manufacturing tolerances, assembly errors, and the like. As a result, it is possible to prevent a problem that the side hole needle 124 affects the cover valve body 170 in the initial state, for example, the tip surface of the side hole needle 124 is pressed against the bottom surface of the insertion recess 172 or reaches the slit 173 whereby the slit 173 of the cover valve body 170 is opened.

Further, in the present practical embodiment, a tip surface 174 of the cover valve body 170 has a centrally protruding shape that protrudes toward the tip side in the central portion rather than in the outer peripheral portion in consideration of the shape of the disc valve 198 of the female connector 190 after being deformed by the insertion of the side hole needle 124 described later, so as to be a substantially mountain-shaped tip surface 174 which is a substantially rotationally symmetric shape around the central axis as a whole.

More specifically, as shown in FIG. 11B, a central portion 174a near the top of the mountain-shaped tip surface 174 has a curved surface that becomes convex toward the tip side by the center of curvature being set closer to the base end side than the tip surface 174. Meanwhile, an outer peripheral portion 174b, which is a region spreading out from the central portion 174a toward the outer periphery to the mountain hem of the mountain-shaped tip surface 174, has an inverted curved inclined surface that becomes concave toward the tip side, which is opposite to the central portion 174a, by the center of curvature being set closer to the tip side than the tip surface 174. The curved surfaces of the central portion 174a and the outer peripheral portion 174b do not have to have a single arcuate shape, but the curvature radii Ra and Rb may be changed in a plurality of stages or gradually. Further, it is desirable that the mountain-shaped tip surface 174 have a surface shape that is smoothly connected without any break points throughout, and it is desirable that both curved surfaces of the central portion 174a and the outer peripheral portion 174b be smoothly connected with a common tangent. It is preferable that the curvature radii of the central portion 174a and the outer peripheral portion 174b on the tip surface 174 are set so that Ra < Rb. Further, it is preferable that the radial width dimension of the outer peripheral portion 174b of the tip surface 174 is larger than the radial dimension of the central portion 174a so that the concave curved surface is provided with a sufficient area as a whole.

Further, a tubular outer peripheral surface 175 of the cover valve body 170 of the present practical embodiment has a cylindrical shape extending from the outer peripheral end of the outer peripheral portion 174b, which is the outer peripheral edge portion of the tip surface 174, toward the axial base end side. In particular, in the present practical embodiment, a stepped surface 175a extending in the radial direction is formed so as to be located in the intermediate portion and on the tip side of the center in the axial direction of the tubular outer peripheral surface 175. The tip side of the stepped surface 175a is a tip side outer peripheral surface 175b having a small diameter, and the base end side of the stepped surface 175a is a base end side outer peripheral surface 175c having a large diameter, so that the entire tubular outer peripheral surface 175 has a stepped cylindrical shape.

The above-mentioned insertion recess 172 extending from the base end surface of the cover valve body 170 toward the tip end side in the axial direction has a depth that extends beyond the stepped surface 175a so as to reach the inner peripheral side of the tip side outer peripheral surface 175b. Alternatively, the depth is set to extend even beyond the tip side outer peripheral surface 175b so as to reach the inner peripheral side of the outer peripheral portion 174b of the tip surface 174. Further, the tip of the side hole needle 124 inserted into the insertion recess 172 may be located on the inner peripheral side of the base end side outer peripheral surface 175c of the cover valve body 170, but in the present practical embodiment, the tip of the side hole needle 124 is located beyond the stepped surface 175a so as to reach the inner peripheral side of the tip side outer peripheral surface 175b.

Further, the cover valve body 170 is assembled in a state of being fitted into the accommodating area 158 of the restraining member 150, and the base end surface of the cover valve body 170 is overlapped with the bottom surface of the accommodating area 158, while the base end side outer peripheral surface 175c of the cover valve body 170 is overlapped with the inner peripheral surface of the accommodating area 158. Further, the stepped surface 175a of the cover valve body 170 is located at substantially the same axial position as the protruding tip surface of the tubular portion 154 projecting into the tip protruding portion 151 of the restraining member 150.

Further, a rigid annular member 176 is externally mounted on the tubular portion 154. The annular member 176 is fixed by being externally fitted onto the tubular portion 154 in a substantially press-fitted state, or by being bonded or welded as necessary. Further, the tip portion of the annular member 176 is bent toward the inner peripheral side, so as to form an annular pressing protrusion 177 protruding as far as the inner peripheral side of the tubular portion 154 on the tip side of the tubular portion 154. Then, the inner surface of the pressing protrusion 177 on the axial base end side is overlapped with the stepped surface 175a of the cover valve body 170, and the protruding tip surface of the pressing protrusion 177 on the inner peripheral side is arranged so as to face the tip side outer peripheral surface 175b of cover valve body 170 in a state of contact or with a gap. The pressing protrusion 177 is located on the stepped surface 175a of the outer peripheral surface 175 of the cover valve body 170, so that the cover valve body 170 is less likely to deform toward the tip end side.

In this way, the annular member 176 is externally fixed to the tubular portion 154 of the restraining member 150, and the pressing protrusion 177 of the annular member 176 covers the outer peripheral portion of the cover valve body 170 from the tip. As a result, the cover valve body 170 is prevented from falling off due to the cover valve body 170 coming out of the restraining member 150, and the tubular portion 154 overlapped with the outer peripheral surface of the cover valve body 170 can also be reinforced.

Further, on the inner peripheral side of the pressing protrusion 177 of the annular member 176, the tip surface 174 of the cover valve body 170 projects toward the tip side in the axial direction. The projecting end (the apex of the central portion 174a) of the tip surface 174 of the cover valve body 170 does not reach the tip of the restraining member 150, but is housed and arranged inside the tip protruding portion 151 of the restraining member 150.

The cover valve body 170 is preferably compressed in the radial direction by the tubular portion 154 and/or the annular member 176. Specifically, in the present practical embodiment, by the outer diameter dimension of the cover valve body 170 on the portion closer to the base end side than the stepped surface 175a (the portion forming the base end side outer peripheral surface 175c) being made larger than the inner diameter dimension of the tubular portion 154 or the like, the base end portion of the cover valve body 170 is compressed in the radial direction. On the other hand, in the tip portion of the cover valve body 170, the outer diameter dimension of the portion forming the tip side outer peripheral surface 175b is slightly smaller than the inner diameter dimension of the pressing protrusion 177. Thus, the portion closer to the tip side than the stepped surface 175a of the cover valve body 170 is configured not to be compressed in the radial direction. As a result, the compressive force in the radial direction is not applied to the portion of the slit 173, and the slit 173 can be prevented from opening unexpectedly. Further, it is preferable that the cover valve body 170 is compressed in the axial direction by the inner peripheral protrusion 156 and the portion of the annular member 176 that is bent toward the inner peripheral side (pressing protrusion 177). As a result, in the initial state described later, the opening 122 of the side hole needle 124 can be more reliably closed by the cover valve body 170. That is, in the present practical embodiment, it can be grasped that in the restraining member 150 that covers the outer periphery of the cover valve body 170, the outer peripheral wall portion that covers the outer periphery of the cover valve body 170 is formed by the tubular portion 154, and the bottom wall portion that covers the bottom surface of the cover valve body 170 is formed by the inner peripheral protrusion 156.

The restraining member 150 having the above structure and the male connector housing 132 having the side hole needle 124 are assembled to each other. That is, the proximal end wall portion 140 of the male connector housing 132 is inserted into each notch 162 provided at the proximal end portion of the restraining member 150 so as to be aligned in the circumferential direction, and by closing the proximal end side opening of each notch 162 with a lid member 178, it is possible to prevent the male connector housing 132 from falling off from the restraining member 150. In other words, by the lid member 178 being attached to the proximal end of the proximal end side protrusion 160 of the restraining member 150, the proximal end side opening of the notch 162 is closed by the lid member 178.

As shown in FIGS. 10 and 13, the lid member 178 of the present practical embodiment is a substantially annular member as a whole. In the lid member 178, at the circumferential positions corresponding to the notches 162 of the restraining member 150, positioning protrusions 180 projecting to the inner peripheral side are provided. Besides, in the lid member 178, at the positions corresponding to the positioning recesses 166 provided at the proximal end portion of the restraining member 150, positioning convex portions 182 are formed. The lid member 178 is externally placed onto the portion of the proximal end portion of the restraining member 150 whose outer diameter dimension is reduced, and the positioning protrusions 180 are inserted into the respective notches 162, while the positioning convex portions 182 are fitted into the respective positioning recesses 166. By so doing, the lid member 178 is attached to the restraining member 150 in a state of being positioned in the axial direction and in the circumferential direction.

The minimum inner diameter dimension of the lid member 178 (inner diameter dimension of the virtual annular surface connecting the inner peripheral surfaces of the positioning protrusions 180) is larger than the maximum outer diameter dimension of the female luer portion 134 (outer diameter dimension at the proximal end side opening). This makes it possible to externally place the lid member 178 onto the proximal end portion of the restraining member 150 in a state where the restraining member 150 and the male connector housing 132 are assembled.

In this way, the lid member 178 is attached to the proximal end portion (proximal end side protrusion 160) of the restraining member 150, and the proximal end side openings of the notches 162 are closed. With this configuration, the restraining member 150 is provided with substantially rectangular guide windows 184 each penetrating inside and outside while extending in the axial direction, and the proximal end wall portions 140 of the male connector housing 132 are inserted into the guide windows 184.

Further, in the assembled state of the restraining member 150 and the male connector housing 132, the side hole needle 124 and the needle hub portion 130 are inserted into the proximal end side protrusion 160, and the side hole needle 124 penetrates the inner peripheral protrusion 156. Furthermore, the tip end portion of the female luer portion 134 is inserted into the proximal end side protrusion 160, and the proximal end portion of the female luer portion 134 projects from the proximal end side protrusion 160 toward the proximal end side.

In the initial state shown in FIGS. 7 to 13, the tip surface 128 of the side hole needle 124 is located in the intermediate portion of the cover valve body 170 in the axial direction (direction of penetration of the slit 173), and the tip surface 128 of the side hole needle 124 is covered with a cover valve body 170. In the present practical embodiment, at the axial position where the opening 122 is formed in the side hole needle 124, the outer periphery of the cover valve body 170 is covered over the entire circumference by the tubular portion 154 of the restraining member 150 and the annular member 176. As a result, the lateral opening (opening 122) of the inner hole 126 of the side hole needle 124 is covered and closed by the cover valve body 170.

A coil spring 186 is provided between the restraining member 150 and the male connector housing 132 that are assembled to each other. The coil spring 186 is an urging means for urging the restraining member 150 toward the tip side with respect to the male connector housing 132. Specifically, the coil spring 186 is externally placed about the needle hub portion 130 and housed in the proximal end side protrusion 160. The tip of the coil spring 186 is fixed to the inner peripheral protrusion 156 of the restraining member 150, while the proximal end of the coil spring 186 is fixed to the tip of the female luer portion 134 and the proximal end wall portions 140, 140.

In the initial state shown in FIGS. 7 to 13, the fitting convex portions 148 provided in the male connector housing 132 are fitted into the respective fitting concave portions 168 provided in the restraining member 150. By means of the concave and convex fitting between the fitting convex portions 148 and the fitting concave portions 168 in the radial direction, the male connector housing 132 and the restraining member 150 are axially engaged with each other, and the axial movement of the restraining member 150 with respect to the male connector housing 132 is restricted. As a result, the lateral opening 122 of the side hole needle 124 is held in a sealed state by the cover valve body 170. That is, in the male connector housing 132, a movement restriction mechanism that engages with the restraining member 150 and holds the cover valve body 170 at the position where the opening 122 is sealed is constituted by including the engagement mechanism between the fitting convex portions 148 and the fitting concave portions 168. The fitting convex portion and the fitting concave portion may be provided on opposite sides of each other, that is, the fitting convex portion may be provided on the restraining member and the fitting concave portion may be provided on the male connector housing.

In the initial state, the restraining member 150 is located at the most distal end side with respect to the male connector housing 132, and the proximal end wall portions 140, 140 of the restraining member 150 are located at the most proximal end side in the respective guide windows 184 so as to be in contact with or slightly separated from the lid member 178. In such an initial state, the coil spring 186 has a substantially natural length or is slightly compressed. Therefore, in the present practical embodiment, a restricting means that restricts movement of the cover valve body 170 toward the tip end is constituted by at least one of the concave and convex fitting between the fitting convex portions 148 and the fitting concave portions 168 and the contact between the proximal end wall portions 140 and the lid member 178 in the guide windows 184. Besides, a positioning means 187, which elastically positions the cover valve body 170 to the tip of the side hole needle 124, is constituted by including the coil spring 186 as an urging means and the above-mentioned restricting means.

Here, the male connector housing 132 is provided with a release mechanism capable of releasing the engagement mechanism between the fitting convex portions 148 and the fitting concave portions 168 as a movement restriction mechanism. Specifically, an operation piece 188 is provided as an operation portion that protrudes to the outer peripheral side continuously from each fitting convex portion 148 provided at the tip of the flexible wall portion 144. The operation piece 188 of the present practical embodiment protrudes from the tip of the flexible wall portion 144 to the outer peripheral side, and curves from the outer peripheral end to the proximal end side to extend toward the proximal end side. In particular, in the present practical embodiment, the operation piece 188 extends in a direction in which the operation piece 188 inclines outwardly toward the proximal end side. As a result, by applying an operating external force to each operation piece 188, specifically, by pressing the proximal end portion of the operation piece 188 toward the inner peripheral side to elastically deform the fitting convex portion 148 and the flexible wall portion 144 so as to be separated to the outer peripheral side, the engagement between the fitting convex portion 148 and the fitting concave portion 168 is released. That is, in the present practical embodiment, the engagement mechanism between the fitting convex portions 148 and the fitting concave portions 168 includes the operation portion (operation piece 188) as the release mechanism.

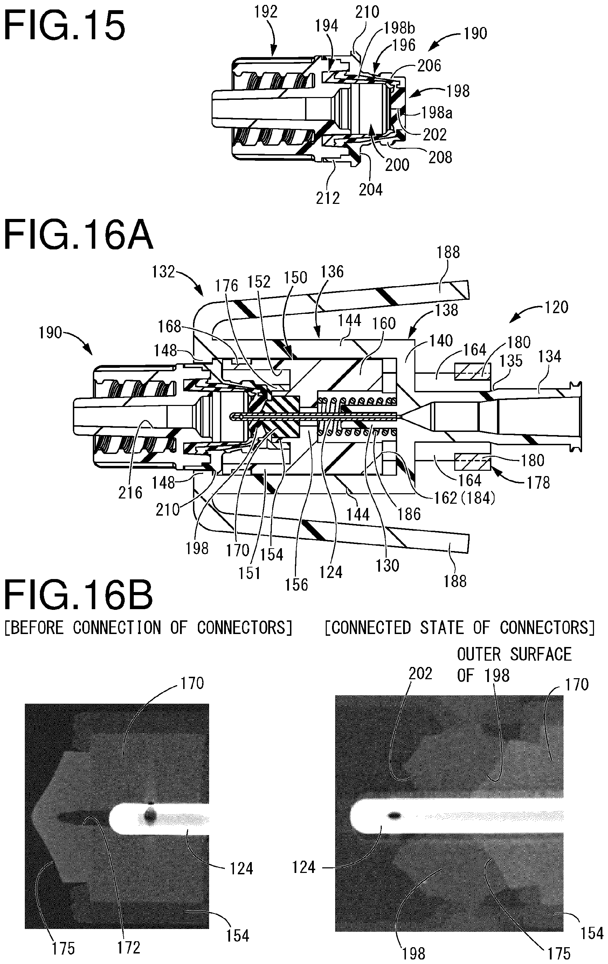

The male connector 120 of the present practical embodiment having the structure as described above in the initial state is connected by inserting the side hole needle 124 into the female connector, which is the connection partner, so that the fluid flow path is in a communicating state. The female connector that can be connected to the male connector 120 of the present practical embodiment is not limited in any way, but a specific example of the female connector is shown in FIG. 15.

A female connector 190 of the present practical embodiment comprises a female connector housing including a base member 192, a lower cover member 194, and an upper cover member 196, and a disc valve 198 as a valve member attached to the female connector housing. That is, the upper and lower cover members 196, 194 are fixed to the base member 192 with the disc valve 198 sandwiched therebetween, and the disc valve 198 is arranged in the opening of the female luer portion 200 constituted by the upper and lower cover members 196, 194.

As the holding structure of the disc valve 198 and its female connector housing, for example, those known in Japanese Unexamined Patent Publication Nos. JP-A-2013-198755, JP-A-2019-72648, and the like can be appropriately adopted. That is, for example, the disc valve 198 has a central portion 198a of circular disk shape extending in the direction perpendicular to the axis with a substantially constant thickness dimension, and is provided with a slit 202 penetrating the central portion 198a in the thickness direction, the slit 202 being closed in the initial state. The shape of the slit 202 is not limited, but various shapes such as a straight line shape, a cross shape, and a radial shape can be adopted. In the present practical embodiment, the slit 202 has a trifurcated shape extending from the center in three directions in the circumferential direction at substantially equal intervals. By forming the slit 202 with a plurality of slits such as a trifurcated slit or increasing its width, the deformability of the disc valve 198 is increased, thereby reducing the insertion resistance and improving the operability. By reducing the width of the slit 202 to the extent that the side hole needle 124 can be inserted, the operability is decreased, but the exposure risk can be suppressed. In order to suppress the exposure risk, the slit may be replaced by a pin hole.

Further, in the disc valve 198, on the outer peripheral side of the central portion 198a, there are formed engaging grooves located on both the inner surface and the outer surface and extending in the circumferential direction. The thickness dimension of the disc valve 198 is made thin by the engaging grooves on the inner and outer surfaces, and an annular or tubular support portion 198b is formed on the outer peripheral side of the thin clasped portion. Under the assembled state to the female connector housing, the support portion 198b is sandwiched between the upper and lower cover members 196, 194, and the inner and outer engaging claws provided on the upper and lower cover members 196, 194 are engaged with the inner and outer engaging grooves of the disc valve 198.

Further, an annular stepped surface 204 is provided on the outer peripheral surface of the upper cover member 196 of the present practical embodiment. The female-connector tip side (right side in FIG. 15), which is the connection tip side with respect to the stepped surface 204, comprises a small-diameter tubular portion 206, and the small-diameter tubular portion 206 is provided with a male screw portion 208 on the outer peripheral surface thereof. The male screw portion 208 makes it possible to connect, for example, a syringe with a lock or the like to the small-diameter tubular portion 206 of the female connector 190. After injecting a drug solution and removing the male connector 120, by connecting the syringe or the like filled with physiological saline or the like and injecting physiological saline or the like into the body of the patient, it is possible to inject the drug solution remaining in the female connector 190 into the patient without being left.

Further, in the female connector housing of the female connector 190, on the outer peripheral surface of the upper cover member 196 constituting the outer peripheral wall on the female-connector tip side, there is provided a flange-shaped portion 210 as an outer protrusion to which the above-mentioned male connector 120 can be engaged in the axial direction. The flange-shaped portion 210 is located closer to the female-connector proximal end side than the male screw portion 208 as the screw portion (left side in FIG. 15), and projects to the outer peripheral side of the male screw portion 208.

In the present practical embodiment, the female connector housing has the stepped surface 204 in the axially intermediate portion, and the female-connector tip side with respect to the stepped surface 204 is a small-diameter portion in which a male screw portion 208 is formed on the outer peripheral surface, while the female-connector proximal end side with respect to the stepped surface 204 is a large-diameter tubular portion 212 having a substantially cylindrical shape with a large diameter. The annular flange-shaped portion 210 projecting to the outer peripheral side is formed at the female-connector tip portion of the large-diameter tubular portion 212. However, the configuration of the flange-shaped portion 210 is acceptable as long as the male connector 120 (in present practical embodiment, the fitting convex portion 148 of the male connector 120) can be engaged in the axial direction. For example, the flange-shaped portion 210 may be formed so as to protrude partially in the circumferential direction, or may be formed so as to be located on the female-connector tip side with respect to the stepped surface 204, or a groove for fitting with the fitting convex portion 148 may be provided instead of the flange-shaped portion 210.

By connecting the male connector 120 to the female connector 190 as described above, the fluid flow path 216 is in a communicating state as shown in FIG. 16. That is, after inserting the small-diameter tubular portion 206 of the female connector 190 into the large-diameter recess 152 of the male connector 120, by the proximal end portions of the operation pieces 188, 188 constituting the release mechanism are pressed toward the inner peripheral side, the flexible wall portions 144 and the fitting convex portions 148 are elastically deformed and/or displaced to displace the fitting convex portions 148 to the outer peripheral side. As a result, the engagement between the fitting convex portions 148 and the fitting concave portions 168 is released, and the restraining member 150 can move in the axial direction with respect to the male connector housing 132.

With respect to the insertion of the small-diameter tubular portion 206 into the large-diameter recess 152, it is acceptable as long as at least a part of the proximal end portion of the small-diameter tubular portion 206 in the axial direction is inserted. Thus, it is not necessary for the small-diameter tubular portion 206 to be inserted across the entire length, or to be inserted until the cover valve body 170 and the disc valve 198 come into contact with each other. This operation is for the purpose of preventing the restraining member 150 from being unintentionally brought into contact with the small-diameter tubular portion 206 and moved to the proximal end side in a state where the fitting convex portions 148 and the fitting concave portions 168 are disengaged. However, it is not essential, and the small-diameter tubular portion 206 may be inserted into the large-diameter recess 152 after the fitting convex portions 148 and the fitting concave portions 168 are disengaged.

Then, in a state where the restraining member 150 is movable with respect to the male connector housing 132, the tip surface of the female connector 190 (the surfaces of the upper cover member 196 and the disc valve 198) and the tip surface inside the large-diameter recess 152 of the restraining member 150 (the surfaces of the annular member 176 and the cover valve body 170) are brought into contact with each other, and the female connector 190 is pushed axially into the male connector housing 132. Due to the pushing force of the female connector 190, the restraining member 150 provided with the cover valve body 170 opposes the urging force of the coil spring 186 (while the coil spring 186 being compressed) and moves to the proximal end side with respect to the side hole needle 124, together with the female connector 190. In the present practical embodiment, the female connector 190 and the restraining member 150 move in a straight line, that is, by a linear translational movement without rotation. For example, by pressing the proximal end portions of the operation pieces 188, 188 toward the inner peripheral side with one hand, the female connector 190 and the restraining member 150 can be moved to the proximal end side with the other hand.

As a result, the side hole needle 124 is inserted through both slits 173, 202 of the cover valve body 170 and the disc valve 198, and the opening 122 of the side hole needle 124 is located within the female luer portion 200 of the female connector 190. By so doing, the flow path of the male connector 120 and the flow path of the female connector 190 are connected with each other to form the fluid flow path 216. As a result, a drug solution such as an anticancer drug enclosed in a bag connected to the female luer portion 134 of the male connector 120 can be injected into the blood vessel of the patient through a catheter or the like communicating with the fluid flow path 216.

In particular, in the present practical embodiment, when the female connector 190 and the restraining member 150 come into contact with each other, the annular member 176 and the upper cover member 196, which are rigid members, come into contact with each other. Thus, the pushing force of the female connector 190 is reliably transmitted by the restraining member 150, thereby stably achieving the movement of the female connector 190 and the restraining member 150 toward the proximal end side.

Further, since the proximal end wall portions 140 of the male connector housing 132 re inserted in the guide windows 184 of the restraining member 150, the axial movement of the restraining member 150 with respect to the male connector housing 132 can be guided by the guide windows 184.

The outer diameter dimension of the restraining member 150 and the inner diameter dimension of the outer tubular portion 136 are substantially equal to each other. Thus, by the outer peripheral surface of the restraining member 150 and the inner peripheral surface of the outer tubular portion 136 being brought into contact with each other as well, the movement of the restraining member 150 with respect to the male connector housing 132 in the direction perpendicular to the axis is prevented, thereby guiding the movement in the axial direction. That is, it can be grasped that the guide means is also formed by the outer peripheral surface of the restraining member 150 and the inner peripheral surface of the outer tubular portion 136, and that the guide portion is formed by the outer peripheral surface of the proximal end side protrusion 160 of the restraining member 150. In particular, the wall portions 146, 146 of the outer tubular portion 136 have larger circumferential dimensions than those of the upper and lower flexible wall portions 144, 144, so that the area of the inner peripheral surface and the deformation strength are sufficiently obtained, thereby improving the guiding action of the restraining member 150 by the outer tubular portion 136 as well.

Further, in the present practical embodiment, the tip surface 174 of the cover valve body 170 of the male connector 120 has a specific shape comprising the central portion 174a having a convex curved shape and the outer peripheral portion 174b having a concave curved shape as described above. Thus, in the connected state of the male connector 120 and the female connector 190, the cover valve body 170 and the disc valve 198 are overlapped with each other with almost no gap. That is, as shown in FIGS. 16A and 16B, when the side hole needle 124 is inserted into the disc valve 198 of the female connector 190, due to the frictional resistance of the side hole needle 124 or the like, the central portion 198a of the disc valve 198 is deformed so as to be recessed inward in the axial direction of the female connector 190, and the outer surface of the central portion 198a of the disc valve 198, which was a flat surface in the initial shape, is deformed so as to be recessed substantially like a bowl.

At this time, as shown in the fluoroscopic image of FIG. 16B, the outer surface of the central portion 198a of the disc valve 198 of the female connector 190 elastically deforms with a curved shape that is convex to the outer surface side from the outer peripheral edge, which is supported by the inner and outer engaging claws of the female connector housing, toward the center. Therefore, since the cover valve body 170 of the male connector 120 has the outer peripheral portion 174b having a concave curved shape on the tip surface 174, when the male connector 120 and the female connector 190 are connected, the outer peripheral portion 174b substantially matches the outer surface of the disc valve 198 that is elastically deformed due to the insertion of the side hole needle 124. Thus, cover valve body 170 and the disc valve 198 can be overlapped in a substantially intimate state with the occurrence of gaps suppressed.

Moreover, whereas the central portion of the disc valve 198 of the female connector 190 is easily pulled inward by being attracted by the surface of the side hole needle 124 to be inserted, since the central portion 174a of the cover valve body 170 of the male connector 120 overlapped thereon has a convex curved shape, good adhesion can be ensured when the male connector 120 and the female connector 190 are connected. As a result, in the connected state of the male connector 120 and the female connector 190, the gap between the overlapping surfaces of the cover valve body 170 and the disc valve 198 is more effectively reduced. Regarding the shape of the slit 202 formed in the disc valve 198, a slit form in which three or more flaps are formed such as a trifurcated shape or a cross shape is preferable to a straight line shape, since the outer peripheral portion 174b having a concave curved shape and the disc valve 198, which elastically deforms with a curved shape, are easily overlapped.

Then, in a state where the flange-shaped portion 210 of the female connector 190 is located closer to the proximal end side than the fitting convex portion 148 of the male connector housing 132, the pressing of the proximal end portions of the operation pieces 188 toward the inner peripheral side is released. Accordingly, the flexible wall portions 144 and the fitting convex portions 148 are elastically restored and deformed, and the fitting convex portions 148 as the connection engaging portions are engaged with the flange-shaped portion 210. As a result, the male connector housing 132 and the female connector 190 are made inseparable and the connected state is maintained.

After injecting the drug solution into the patient, the fluid flow path 216 is shut off by disconnecting the male connector 120 and the female connector 190. That is, by applying an operating external force to the operation pieces 188, specifically, by pressing the proximal end portions of the operation pieces 188 toward the inner peripheral side to displace the fitting convex portions 148 toward the outer peripheral side, the fitting convex portions 148 and the flange-shaped portion 210 are disengaged, so that the female connector 190 becomes separable from the male connector housing 132. That is, in the present practical embodiment, by operating the operation pieces 188 that release the engagement between the fitting convex portions 148 and the fitting concave portions 168, the engagement between the fitting convex portions 148 and the flange-shaped portion 210 is also configured to be released. By grasping the female connector 190 in this state and pulling it toward the tip side (left side in FIG. 16A), the side hole needle 124 is removed from the disc valve 198 of the female connector 190, and the female connector 190 is restored to the state shown in FIG. 15.

Further, due to the female connector 190 being pulled out to the tip side (left side in FIG. 16A), the coil spring 186 is elastically restored and deformed, and the restraining member 150 moves to the tip side with respect to the male connector housing 132. As a result, the tip of the side hole needle 124 is covered with the cover valve body 170, and by releasing the pressing force on the operation pieces 188, the fitting convex portions 148 and the fitting concave portions 168 are engaged, so that the restraining member 150 becomes immovable with respect to the male connector housing 132. As a result, exposure or leakage of the drug solution from the opening 122 of the side hole needle 124 can be more reliably prevented.

In the male connector 120 of the present practical embodiment having the above structure as well, the positioning means 187 for positioning the cover valve body 170 to the tip of the side hole needle 124 is provided, and the rigid restraining member 150 and annular member 176 for covering the outer periphery of the cover valve body 170 are provided. Thus, as in the preceding first practical embodiment, when the male connector 120 is disconnected from the female connector 190, the opening 122 of the side hole needle 124 is more reliably sealed by the cover valve body 170, thereby more reliably preventing exposure or leakage of the drug solution. In particular, in the present practical embodiment, there is provided the movement restriction mechanism for restricting the movement of the restraining member 150 by the restraining member 150 and the male connector housing 132 being engaged. Thus, the sealed state of the opening 122 of the side hole needle 124 can be stably maintained.

Further, the male connector 120 of the present practical embodiment is provided with the release mechanism (operation pieces 188) for releasing the engagement between the restraining member 150 and the male connector housing 132, and is configured such that the movement operation of the restraining member 150 and the female connector 190 toward the proximal end side and the disengaging operation by means of the release mechanism (operation pieces 188) are performed separately. As a result, the disengagement of the restraining member 150 and the male connector housing 132 can be intentionally performed, and the stability and reliability of the disengaging operation can be improved. In this way, the releasing operation of the movement restriction mechanism (the operation of pressing the operation pieces 188 toward the central axis) and the movement operation of the restraining member (the operation of moving the restraining member 150 along the central axis) are made separate operations, so that the operating force required for each operation can be set separately. With this configuration, the certainty of the movement restriction of the restraining member by means of the movement restriction mechanism and the good movement operability of the restraining member when the movement restriction mechanism is released can be achieved at the same time. Specifically, for example, it is possible to increase the engaging force between the restraining member 150 and the male connector housing 132 while ensuring good movement operability of the restraining member 150. Therefore, it is also possible to achieve both the improvement of the operability of the movement operation and the improvement of the operability of the disengaging operation.

In the present practical embodiment, in the initial state, the tip protruding portion 151 of the restraining member 150 is restricted from moving in a state of protruding toward the tip side with respect to the cover valve body 170. This makes it possible to prevent the user from unintentionally touching the cover valve body 170 so that the opening 122 of the side hole needle 124 is opened, thereby preventing exposure or leakage of a drug solution such as an anticancer drug.

Further, in the present practical embodiment, since the restraining member 150 is moved to the proximal end side according to the guide means, the restraining member 150 can be moved while suppressing rattling with respect to the male connector housing 132. It would also be acceptable that, for example, the male connector housing 132 is provided with a groove extending in the axial direction, and the restraining member 150 is provided with a guide piece projecting to the outer peripheral side, so as to provide the guide means by the guide piece sliding within the groove.

Furthermore, in the present practical embodiment, the cover valve body 170 projects toward the tip side, and has a shape that substantially corresponds to the disc valve 198 of the female connector 190 that is deformed so as to be pulled toward the tip side when the side hole needle 124 is inserted. As a result, when the male connector 120 and the female connector 190 are connected, the cover valve body 170 and the disc valve 198 come into contact with each other with almost no gap, and when the side hole needle 124 is removed from the female connector 190, it is possible to prevent exposure or leakage of the drug solution that has entered the gap between the cover valve body 170 and the disc valve 198.

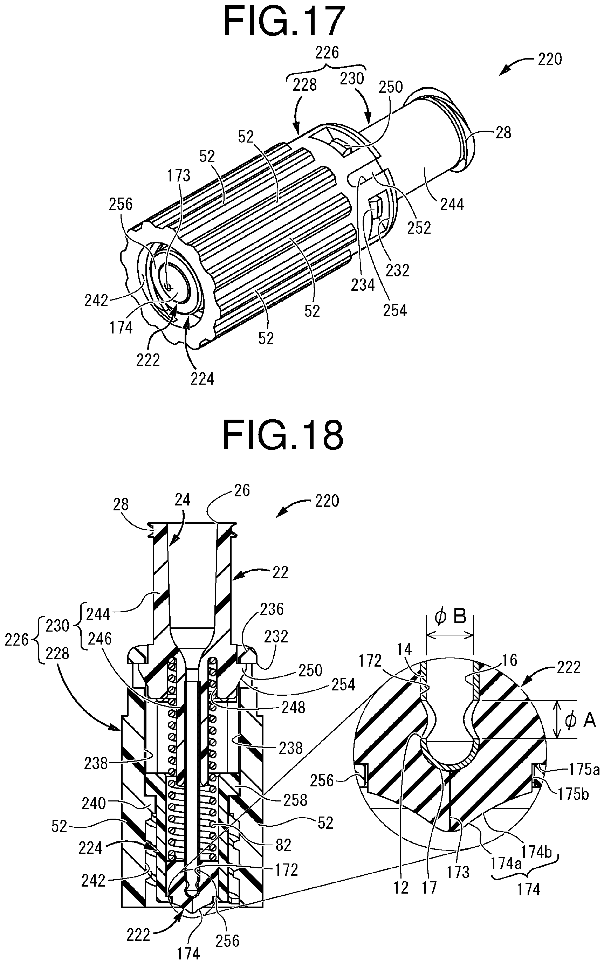

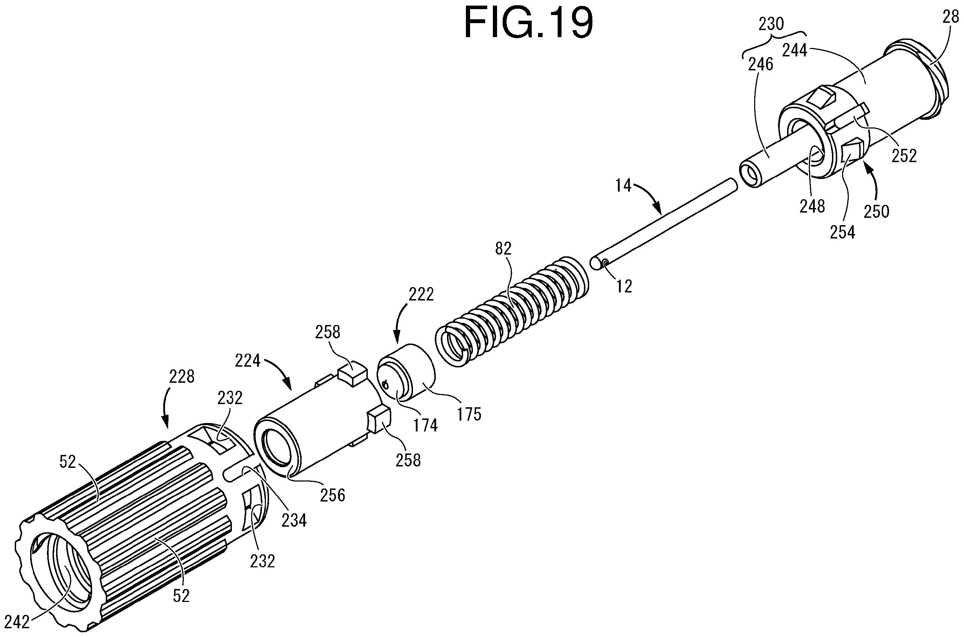

Next, FIGS. 17 and 18 show a male connector 220 as a third practical embodiment of the present invention. The male connector 220 of the present practical embodiment has a similar structure to that of the male connector 10 of the first practical embodiment. That is, as shown in FIG. 19, the male connector 220 of the present practical embodiment includes the side hole needle 14 having the opening 12 opened sideways at the tip of the hollow needle, a cover valve body 222 that covers the opening 12, a rigid restraining member 224 that covers the outer periphery of the cover valve body 222, a male connector housing 226 that covers the outer periphery of the restraining member 224, and the coil spring 82 as an urging means that urges the restraining member 224 from the proximal end side to the tip side. In the present practical embodiment, the axial direction means the vertical direction in FIG. 18 which is the central axial direction of the side hole needle 14. Further, the distal end side or tip side refers to the lower side in FIG. 18 which is the side where the opening 12 is provided in the side hole needle 14, while the proximal end side or base end side refers to the upper side in FIG. 18 which is the side where the side hole needle 14 is fixed. Further, in the following description, the members and parts substantially the same as those of the preceding practical embodiment are designated by the same reference numerals as those of the preceding practical embodiment in the drawings, and detailed description thereof will be omitted.

In the preceding first practical embodiment, the male connector housing 18 is a single member formed by integral molding, but the male connector housing 226 of the present practical embodiment has a structure in which a plurality of members are axially connected. That is, in the male connector housing 226 of the present practical embodiment, a tubular peripheral wall portion 228 that covers the outer periphery of the restraining member 224 and a needle hub 230 that supports the side hole needle 14 have a structure that is separate from each other, and the tubular peripheral wall portion 228 is connected to the tip side of the needle hub 230.

The tubular peripheral wall portion 228 has a substantially tubular shape as a whole, and the proximal end portion thereof includes through windows 232 each penetrating in the thickness direction of the peripheral wall and having a substantially rectangular shape in plan view, and positioning recesses 234 each penetrating in the thickness direction of the peripheral wall and extending from the proximal end to the tip side with a certain degree of axial dimension. In the present practical embodiment, four through windows 232 are formed at substantially equal intervals in the circumferential direction, and a pair of positioning recesses 234 facing each other in the diametrical direction are formed at positions away from the through windows 232 in the circumferential direction. By providing the positioning recess 234, the peripheral wall at the proximal end of the tubular peripheral wall portion 228 can be elastically deformed in the radial direction. In the present practical embodiment, the inner peripheral surface of the proximal end of the tubular peripheral wall portion 228 is provided with tapered surfaces 236 that gradually increase in diameter toward the proximal end side at circumferential positions corresponding to the through windows 232.

Further, the inner peripheral surface of the proximal end portion of the tubular peripheral wall portion 228 is provided with portions whose inner diameter dimensions are increased partially in the circumferential direction, and inner peripheral groove portions 238 are provided by the said portions where the inner diameter dimensions are increased. The inner peripheral groove portions 238 each have a substantially rectangular cross section, and extend from the proximal end to the tip side with a certain degree of axial dimension. In other words, an annular inner peripheral protrusion 240 that protrudes toward the inner peripheral side is provided at the axially intermediate portion of the tubular peripheral wall portion 228, and the inner peripheral groove portions 238 that extend in the axial direction are formed at the proximal end side of the inner peripheral protrusion 240. In the present practical embodiment, four inner peripheral groove portions 238 are provided and are formed at substantially equal intervals in the circumferential direction.

Further, a female screw portion 242 is formed on the inner peripheral surface of the tip portion of the tubular peripheral wall portion 228. The female screw portion 242 can be screwed with the male screw portion 102 of the female connector 86 which is the connection partner.

The needle hub 230 is integrally provided with a female luer portion 244 to which an external flow path is connected and a needle hub portion 246 that substantially holds the side hole needle 14. Both the female luer portion 244 and the needle hub portion 246 have a substantially tubular shape, and the needle hub portion 246 has a smaller diameter than the female luer portion 244. The needle hub portion 246 continuously extends from the inner peripheral side of the female luer portion 244 toward the tip side. That is, the axially intermediate portion of the needle hub 230 has a double-cylinder structure in which the female luer portion 244 is externally disposed about the needle hub portion 246, and an annular recess 248 that opens to the tip side is formed radially between the female luer portion 244 and the needle hub portion 246.

Further, engaging protrusions 250 protruding to the outer peripheral side are provided on the outer peripheral surface of the tip portion of the female luer portion 244. Each engaging protrusion 250 has a shape substantially corresponding to the through window 232, and has a substantially rectangular shape in a plan view. In the present practical embodiment, four engaging protrusions 250 are provided and are formed at positions corresponding to the through windows 232 in the circumferential direction. Further, at positions away from the engaging protrusions 250 in the circumferential direction, positioning convex portions 252 projecting to the outer peripheral side and extending in the axial direction are provided. In the present practical embodiment, the positioning convex portions 252 are formed at the positions corresponding to the positioning recesses 234 in the circumferential direction. The tip surface of each engaging protrusion 250 is a tapered surface 254 whose protruding dimension toward the outer peripheral side gradually decreases toward the tip side.

Further, the restraining member 224 has a substantially tubular shape as a whole, and an annular pressing protrusion 256 projecting to the inner peripheral side is provided at the tip, while outer peripheral protrusions 258 projecting to the outer peripheral side are provided at the proximal end. The outer peripheral protrusions 258 each have a rectangular block-shaped protrusion with a size substantially corresponding to the inner peripheral groove portion 238, and are formed at positions corresponding to the inner peripheral groove portions 238 in the circumferential direction.

Furthermore, the cover valve body 222 of the present practical embodiment has a similar shape to that of the cover valve body 170 of the second practical embodiment as a whole, but in the present practical embodiment, the bottom surface of the insertion recess 172 is located at the axial position that is substantially equal to that of the stepped surface 175a, and at the center of the tip portion of the cover valve body 222, the slit 173 is formed across the entire length on the tip side of the stepped surface 175a. As a result, the tip surface 17 of the side hole needle 14 and the bottom surface of the insertion recess 172 are in contact with each other in a state of a so-called zero touch, or the tip of the side hole needle 14 slightly enters the slit 173. As a result, almost no space is provided between the tip surface 17 of the side hole needle 14 and the bottom surface of the insertion recess 172. Further, also in the present practical embodiment, the outer diameter dimension at the tip portion of the cover valve body 222 is slightly smaller than the inner diameter dimension of the pressing protrusion 256, so that the portion of the slit 173 in the cover valve body 222 is not compressed in the radial direction.

It is preferable that the outer diameter dimension of the cover valve body 222 (outer diameter dimension of the base end side outer peripheral surface 175c) is substantially equal to or slightly larger than the inner diameter dimension of the restraining member 224, and it is preferable that when the cover valve body 222 and the restraining member 224 are assembled, the cover valve body 222 is slightly compressed in the radial direction by the inner peripheral surface of the restraining member 224. Further, it is preferable that the inner diameter dimension of the cover valve body 222 (inner diameter dimension of the insertion recess 172) is substantially equal to or slightly smaller than the outer diameter dimension of the side hole needle 14, and when the side hole needle 14 is inserted into the cover valve body 222, the inner peripheral surface of the cover valve body 222 (inner peripheral surface of the insertion recess 172) is configured to be pressed against the tip of the side hole needle 14. It would also be acceptable to provide an annular convex portion on the outer surface of the cover valve body 222 so as to be slightly compressed in the radial direction by the inner peripheral surface of the restraining member 224. Further, particularly strong compression may be generated in an annular shape on the tip side and the proximal end side of the opening 12 of the side hole needle 14. Further, an annular convex portion may be provided on the outer peripheral surface of the side hole needle 14 and/or the inner peripheral surface of the restraining member 224.

The restraining member 224 to which the cover valve body 222 is attached is inserted from the proximal end of the tubular peripheral wall portion 228. At that time, the outer peripheral protrusions 258 of the restraining member 224 are fitted into the respective inner peripheral groove portions 238 of the tubular peripheral wall portion 228, so that the restraining member 224 cannot rotate about the central axis with respect to the tubular peripheral wall portion 228 but is movable in the axial direction. Then, the inner peripheral protrusion 240 of the tubular peripheral wall portion 228 and the outer peripheral protrusions 258 of the restraining member 224 are in contact with each other, so as to restrict movement of the restraining member 224 toward the tip side with respect to the tubular peripheral wall portion 228.

The needle hub 230 to which the side hole needle 14 is fixed is inserted from the proximal end side of the tubular peripheral wall portion 228. That is, the needle hub 230 is inserted from the proximal end side opening of the tubular peripheral wall portion 228 in a state where the through windows 232 of the tubular peripheral wall portion 228 and the engaging protrusions 250 of the needle hub 230 are aligned in the circumferential direction, so that the positioning convex portions 252 enter the respective positioning recesses 234 to prevent the relative rotation between the tubular peripheral wall portion 228 and the needle hub 230, and to restrict movement of the needle hub 230 toward the tip side with respect to the tubular peripheral wall portion 228. Further, by the engaging protrusions 250 entering and being engaging with the through windows 232 as well, the relative rotation between the tubular peripheral wall portion 228 and the needle hub 230 is prevented, and movement of the needle hub 230 toward the proximal end side with respect to the tubular peripheral wall portion 228 is restricted.

As a result, the tubular peripheral wall portion 228 and the needle hub 230 are assembled in a state of being positioned in the axial direction. When assembling the tubular peripheral wall portion 228 and the needle hub 230, the tapered surfaces 236 at the proximal end of the tubular peripheral wall portion 228 and the tapered surfaces 254 at the tips of the engaging protrusions 250 come into contact with each other, so that the proximal end of the tubular peripheral wall portion 228 is easily deformed toward the outer peripheral side.

In the male connector 220 of the present practical embodiment having the above-mentioned structure as well, by the female connector 86 as shown in FIG. 5, for example, being connected from the tip side by screw feed, the tip surface 174 of the cover valve body 222 and the base end surface of the valve member 94 (upper surface in FIG. 5) is brought into contact with each other, whereby the side hole needle 14 is inserted through the cover valve body 222 and the valve member 94.

In the present practical embodiment, since the male connector housing 226 is constituted by connecting the tubular peripheral wall portion 228 and the needle hub 230, which are mutually separate elements, the inner peripheral protrusion 240 and the outer peripheral protrusions 258 can be easily provided inside the male connector housing 226. Further, the inner peripheral protrusion 240 and the outer peripheral protrusions 258 are provided inside the tubular peripheral wall portion 228, thereby preventing, for example, the outer peripheral protrusions 258 from being pushed toward the inner peripheral side from the outside to be unintentionally disengaged from the inner peripheral protrusion 240.

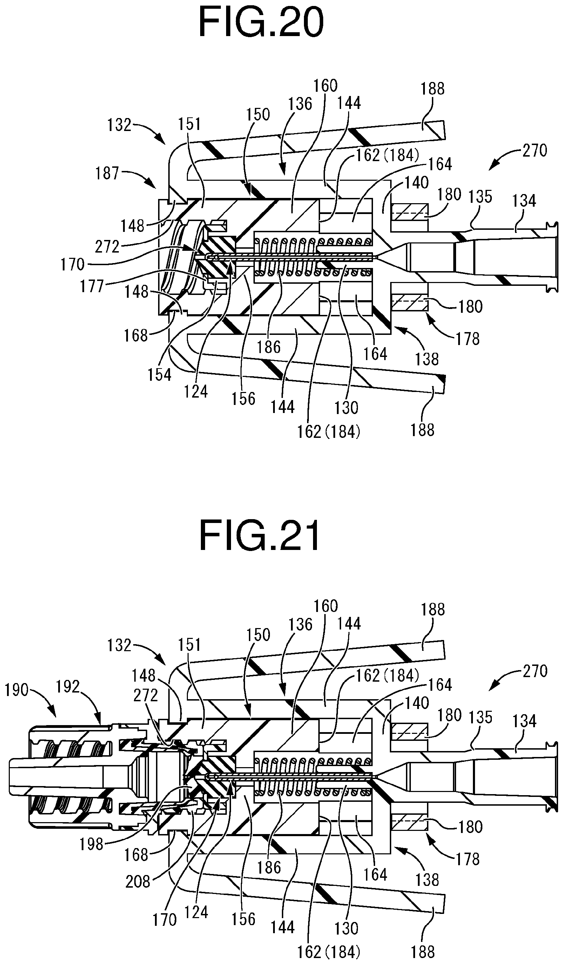

Next, FIG. 20 shows a male connector 270 as a fourth practical embodiment of the present invention. The male connector 270 of the present practical embodiment has a similar structure to that of the male connector 120 of the preceding second practical embodiment. The difference between the male connector 270 of the present practical embodiment and the male connector 120 of the second practical embodiment is that in male connector 270 of the present practical embodiment, the inner peripheral surface of the tip protruding portion 151 of the restraining member 150 is provided with a female screw portion 272 to be screwed with the male screw portion 208 of the female connector 190 (see FIG. 15), which is the connection partner. Besides, as the cover valve body, the cover valve body 222 having the shape of the third practical embodiment is adopted. In the present practical embodiment, the axial direction means the left-right direction in FIG. 20, which is the central axial direction of the side hole needle 124. Further, the distal end side or tip side refers to the left side in FIG. 20 on which the opening 122 is provided in the side hole needle 124, while the proximal end side or base end side refers to the right side in FIG. 20 on which the side hole needle 124 is fixed.

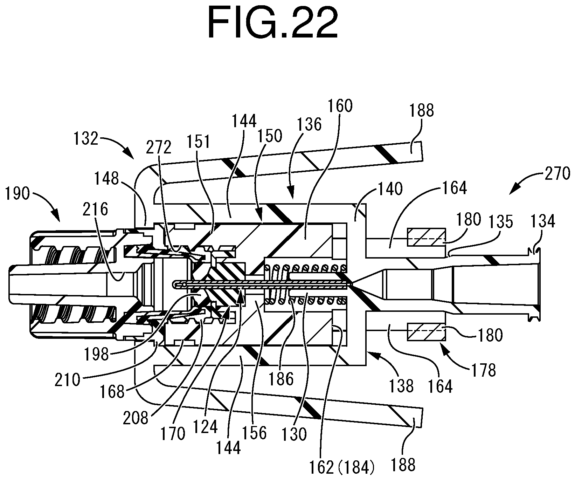

Hereinafter, a specific example of the connection method between the male connector 270 and the female connector 190 of the present practical embodiment will be described with reference to FIGS. 21 and 22, but the connection method between the male connector 270 and the female connector 190 is not limited to the following method.

First, as shown in FIG. 21, the male connector 270 and the female connector 190 are brought close to each other, and the female screw portion 272 and the male screw portion 208 are screwed together. Then, by moving the female connector 190 in the direction of approach to the male connector 270 by screw feed, the small-diameter tubular portion 206 of the female connector 190 enters the tip protruding portion 151. The movement of the female connector 190 with respect to the male connector 270 can be restricted by, for example, the pressing protrusion 177 of the male connector 270 and the proximal end surface (right end surface in FIG. 21) of the small-diameter tubular portion 206 coming into contact with each other, or by the tip surface of the tip protruding portion 151 and the stepped surface 204 of the female connector 190 coming into contact with each other. That is, in the present practical embodiment, the connecting portion provided on the male connector 270 and connected to the female connector 190 is constituted by the female screw portion 272. In particular, in the present practical embodiment, such a connecting portion (female screw portion 272) is provided on the restraining member 150 of the male connector 270, so that the female connector 190 and the male connector 270, particularly the restraining member 150, are connected to each other.

In the present practical embodiment, at the moving end of the female connector 190, the pressing protrusion 177 and the proximal end surface of the small-diameter tubular portion 206 are in contact with each other. As a result, the tip surface 174 of the cover valve body 222 in the male connector 270 and the base end surface of the disc valve 198 as a valve member in the female connector 190 are overlapped and protrude with respect to each other. It is also possible to make the pressing protrusion 177 smaller than the small-diameter tubular portion 206 so that the pressing protrusion 177 is inserted into the small-diameter tubular portion 206.

From this state, the operation pieces 188 are operated (operation pieces 188 are pressed toward the inner peripheral side) to disengage the fitting convex portions 148 and the fitting concave portions 168, and as shown in FIG. 22, the male connector housing 132 is moved toward the tip side with respect to the restraining member 150 and the female connector 190 (or the restraining member 150 and the female connector 190 are moved to the proximal end side with respect to the male connector housing 132) against the urging force of the coil spring 186 (while compressing the coil spring 186). As a result, the side hole needle 124 is inserted through the cover valve body 222 and the disc valve 198, and the fluid flow path 216 is in a communicating state.

However, the method of connecting the male connector 270 and the female connector 190 is not limited to the above method. For example, it would also be acceptable that, after the operation pieces 188 are operated to disengage the fitting convex portions 148 and the fitting concave portions 168, the female screw portion 272 and the male screw portion 208 are screwed to connect the male connector 270 and the female connector 190, and then the male connector housing 132 is moved to the tip side to insert the side hole needle 124 through the cover valve body 222 and the disc valve 198 while the fitting convex portions 148 and the flange-shaped portion 210 are engaged with each other.

In the male connector 270 of the present practical embodiment, before the side hole needle 124 is inserted through the cover valve body 222 and the disc valve 198, the female screw portion 272 and the male screw portion 208 are screwed together so that the tip surface of the cover valve body 222 and the base end surface of the disc valve 198 are brought into contact with each other. That is, the side hole needle 124 is inserted and removed in a state where the cover valve body 222 and the disc valve 198 are overlapped with each other. Thus, during insertion and removal of the side hole needle 124, a gap is prevented from being generated between the cover valve body 222 and the disc valve 198. By so doing, leakage or exposure of the drug solution through the gap can be effectively avoided.

Further, when the male connector housing 132 is moved, the restraining member 150 and the female connector 190 are connected to each other. Thus, during engaging the fitting convex portions 148 with the flange-shaped portion 210, the female connector 190 does not unintentionally disengage from the restraining member 150, thereby more reliably performing the engagement operation between the fitting convex portions 148 and the flange-shaped portion 210, that is, the operation of moving the male connector housing 132 to insert the side hole needle 124 through the cover valve body 222 and the disc valve 198.

In particular, by screwing the female screw portion 272 and the male screw portion 208, the central axes of the male connector 270 and the female connector 190 are aligned with each other, and the side hole needle 124 can be more reliably inserted through the slit 202 at the center of the disc valve 198. As a result, unintended deformation of the disc valve 198 can also be suppressed, thereby preventing generation of a gap between the cover valve body 170 and the disc valve 198 that causes leakage or exposure of the drug solution.

Although the practical embodiments of the present invention have been described above, the present invention is not limitedly interpreted by the specific description in the practical embodiments, but may be embodied with various changes, modifications and improvements which may occur to those skilled in the art.

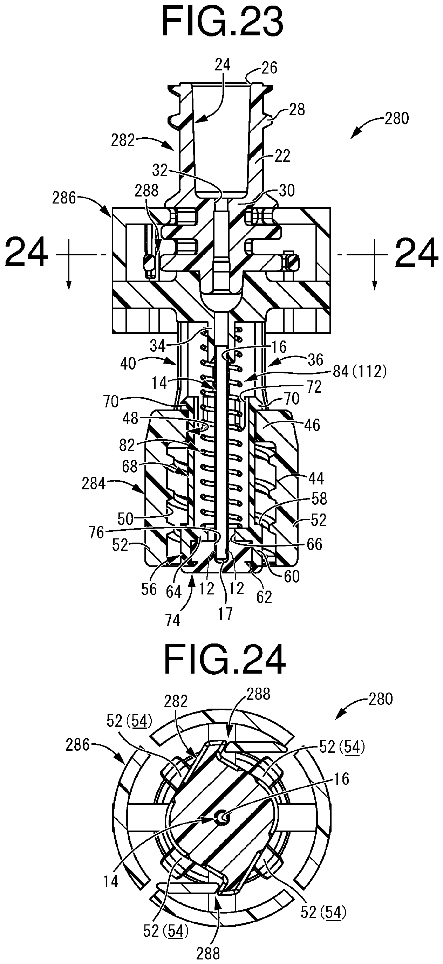

For example, in the preceding first practical embodiment, in the male connector 10 (male connector housing 18), the female luer portion 20, the outer tubular portion 36, and the lock portion 42 are integrally formed, and when the male connector 10 is screwed with the female connector 86, the entire male connector 10 is configured to rotate about the central axis with respect to the female connector 86. Further, in the preceding third practical embodiment, in the male connector housing 226, the tubular peripheral wall portion 228 and the needle hub 230 are formed separately and cannot rotate. However, the present invention is not limited to these embodiments. Specifically, as in a male connector 280 shown in FIGS. 23 and 24, a female luer portion 282 and a lock portion 284 may be formed as separate elements so as to be relatively rotatable around the central axis.

In the alternative embodiment shown in FIGS. 23 and 24, a tubular body 286 is fixed to the proximal end side of the lock portion 284, and the tip portion of the female luer portion 282 is inserted into the tubular body 286 so as to be relatively rotatable. A ratchet mechanism 288 is formed between the female luer portion 282 and the tubular body 286.

In the ratchet mechanism 288, when the female luer portion 282 is rotated clockwise in FIG. 24, the protrusions of the female luer portion 282 shown in the upper and lower portions in FIG. 24 abut on the tip surfaces of the flexible pieces projecting within the tubular body 286 and are prevented from rotating by the tubular body 286. Therefore, the clockwise rotational force applied to the female luer portion 282 is directly transmitted from the tubular body 286 to the lock portion 284, and the female luer portion 282, the tubular body 286, and the lock portion 284 rotate together.

On the other hand, when the female luer portion 282 is rotated counterclockwise in FIG. 24, the protrusions of the female luer portion 282 hit the tip portions of the flexible pieces of the tubular body 286 from the side, so that the flexible pieces elastically deform so as to escape, thereby allowing relative rotation of the female luer portion 282 with respect to the tubular body 286. Therefore, the counterclockwise rotational force applied to the female luer portion 282 is not transmitted to the tubular body 286 or the lock portion 284, and the female luer portion 282 and the tubular body 286 idle.

Therefore, when connecting the female luer portion 282 with a connector with a lock of a drug solution flow path extending from a drug solution bag or the like, it is possible to rotationally lock the connector to be connected with the female luer portion 282 in a state where the female luer portion 282 is prevented from rotating clockwise by grasping the tubular body 286 and the lock portion 284.

On the other hand, when the connector with a lock is connected to the female luer portion 282, the connector is prevented from being unintentionally detached from the female luer portion 282. That is, even when a rotational force in the direction of detaching the connector from the female luer portion 282 is applied from the connector side with the lock or the catheter side connected to the lock portion 284, due to the function of the ratchet mechanism 288, the action of the rotational force in the direction of detaching the connector from the female luer portion 282 is avoided, thereby stably maintaining the screwed state of the connector with the male screw portion 28 of the female luer portion 282.

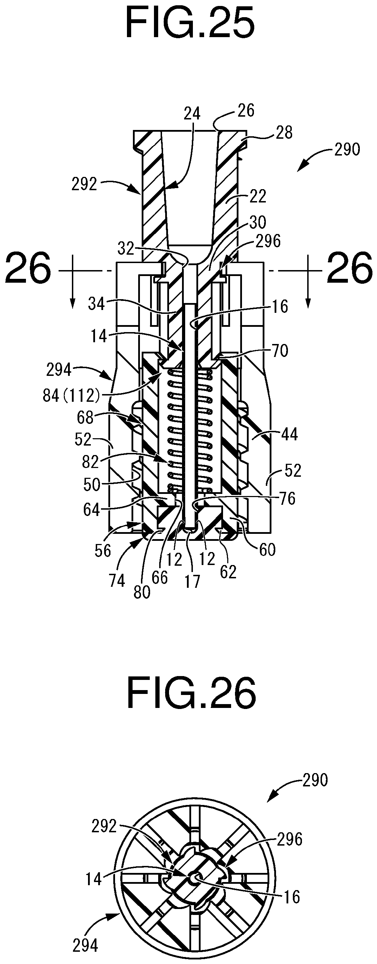

Similarly, in a male connector 290 shown in FIGS. 25 and 26, a member constituting a female luer portion 292 and a member constituting a lock portion 294 are separate elements, and a ratchet mechanism 296 is formed between the two members. The male connector 290 of the present practical embodiment having such a structure can also exhibit a similar effect to that of the male connector 280 shown in FIGS. 23 and 24.

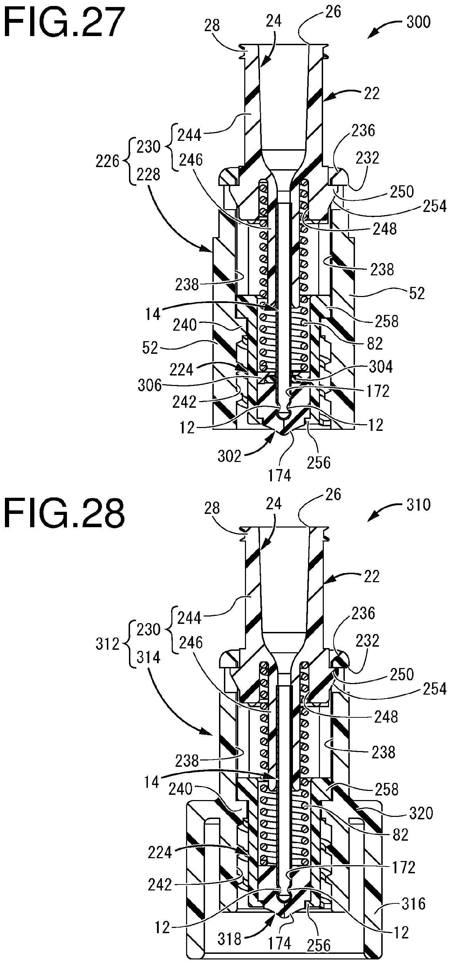

Further, as in a male connector 300 shown in FIG. 27, at the base end surface of a cover valve body 302, an O-ring 304 as an annular sealing portion that is externally attached to the side hole needle 14, and a limiting member 306 for limiting the deformation of the base end surface of the cover valve body 302 may be provided. In the present embodiment, the O-ring 304 is provided on the inner peripheral side of the base end surface of the cover valve body 302, and the annular limiting member 306 is provided so as to cover the base end side from the outer peripheral side of the O-ring 304. As a result, the limiting member 306 and the outer peripheral portion of the base end surface of the cover valve body 302 are in contact with each other. The limiting member 306 can be attached to the restraining member 224, the side hole needle 14, or the like by being inserted into the restraining member 224 in a press-fitted state or fixed by adhesion, welding, or the like. Then, by the O-ring 304 being compressed radially between the limiting member 306 and the side hole needle 14, a sealing is provided between the cover valve body 302 and the outer peripheral surface of the side hole needle 14. In the present embodiment, the tip of the coil spring 82 is fixed to the base end surface of the limiting member 306.

By providing the O-ring 304 as described above, even if a gap is generated between the side hole needle 14 and the cover valve body 302 due to insertion and removal of the side hole needle 14 with respect to the cover valve body 302 or the like, leakage or exposure of the drug solution through the gap can be prevented. Further, by providing the limiting member 306 that is in contact with the base end surface of the cover valve body 302, the deformation of the base end surface of the cover valve body 302 is suppressed, thereby preventing generation of a gap between the cover valve body 302 and the side hole needle 14.

In the present embodiment, the O-ring 304 and the limiting member 306 are provided at the same time, but these may be provided independently. When an annular sealing portion is provided on the base end side of the cover valve body, the sealing portion does not need to be an O-ring. For example, it would also be acceptable to provide a tubular portion integrally protruding from the cover valve body toward the base end side so as to be externally placed onto the side hole needle, and to externally fasten an annular member onto the side hole needle so as to tighten the tubular portion. In such a case, it is preferable to provide an annular recess on the inner peripheral surface of the tubular portion so that the tubular portion is in contact with the outer peripheral surface of the side hole needle in a state of linear contact. As a result, it is possible to avoid an increase in frictional resistance with the sealing portion when the side hole needle is inserted and removed.

When the limiting member is provided alone, the limiting member may partially cover the base end surface of the cover valve body as in the present embodiment, or for example, may be a substantially annular member that covers substantially the entire surface of the base end surface of the cover valve body. The limiting member is preferably formed of a material harder than the cover valve body, and more preferably formed of, for example, a metal or a hard synthetic resin.

Further, in the preceding first and third practical embodiments, the tip of the cover valve body 74, 222 projects toward the tip side from the male connector housing 18, 226 to be exposed to the outside. However, like a male connector 310 shown in FIG. 28, it would also be acceptable to provide a cover portion 316 protruding from a tubular peripheral wall portion 314 of a male connector housing 312 toward the tip side so that the tip end of a cover valve body 318 is covered with the cover portion 316. The cover portion 316 of the present embodiment has a diameter larger than that of the tubular peripheral wall portion 314, and the proximal end of the cover portion 316 is connected by an annular connecting wall portion 320 at the axially intermediate portion of the tubular peripheral wall portion 314. This prevents the user from unintentionally touching the cover valve body 318 to cause leakage or exposure of the drug solution from the opening 12 of the side hole needle 14. Besides, when connecting the female connector 86, contact between the cover portion 316 and the female connector 86 can be avoided, thereby exhibiting good operability. The cover portion 316 may be separable from the tubular peripheral wall portion 314.

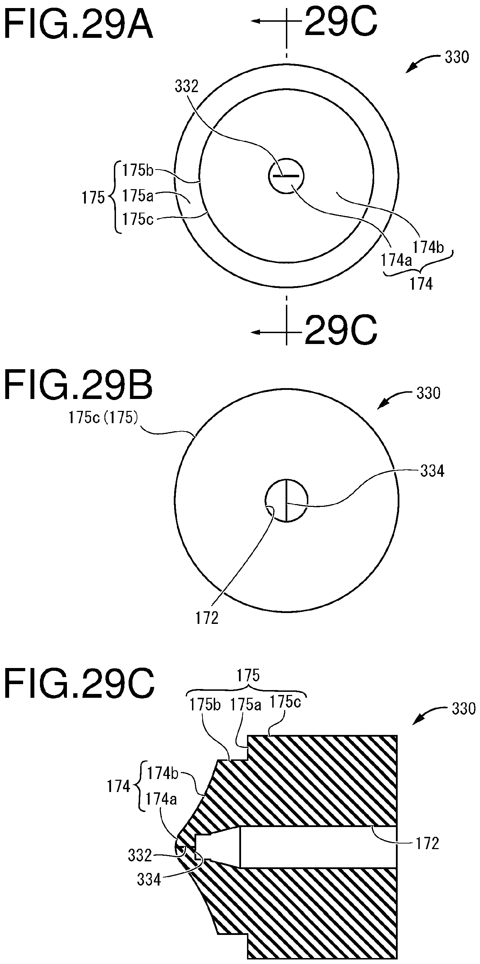

Furthermore, in the male connector according to the present invention, a cover valve body 330 shown in FIGS. 29A to 29C can also be adopted. That is, the cover valve body 330 is provided with a tip side slit 332 and a base end side slit 334, and the directions of extension of these slits 332, 334 are mutually different. In particular, in the present embodiment, both slits 332, 334 extend in the directions orthogonal to each other. This can effectively prevent leakage and exposure of the drug solution. In the present embodiment, the tip and the base end side slits 332, 334 are formed on the tip side of the cover valve body 330 with respect to the insertion recess 76, so as to connect the tip surface 174 and the insertion recess 76 with each other. Further, the cover valve body in which the directions of extension of the slits are different between the tip side and the base end side may be formed by, for example, a plurality of elastic bodies whose slits have mutually different directions of extension being overlapped in the axial direction, or by inserting cutters in different directions from the tip end side and the base end side with respect to a single cover valve body so as to form slits extending in different directions. The width dimensions of the tip side slit and the base end side slit may be different from each other.

Further, in the preceding first practical embodiment, in the tip portion of the restraining member 56, the outer peripheral wall portion 60 is formed over the entire circumference in the circumferential direction, but may alternatively be partially formed on the circumference. When the outer peripheral wall portion is partially formed on the circumference, it is preferable that the outer peripheral wall portion is formed at a position corresponding to the opening of the side hole needle on the circumference. However, the outer peripheral wall portion may be formed at a position that does not correspond to the opening of the side hole needle. Similarly, in the preceding first practical embodiment, the bottom wall portion 64 is formed in an annular shape over the entire circumference at the tip portion of the restraining member 56, but may alternatively be partially formed on the circumference. The bottom wall portion of the restraining member is not essential. Further, as shown in FIG. 27, by providing the limiting member on the base end surface of the cover valve body, it is possible to grasp the limiting member as the bottom wall portion. That is, the bottom wall portion may be formed separately from the restraining member.

Further, in the preceding first to fourth practical embodiments, the insertion recess 76, 172 into which the tip of the side hole needle 14, 124 is inserted is formed on the base end side end surface of the cover valve body 74, 170, 222. However, the present invention is not limited to this embodiment. That is, a slit may be adopted instead of the insertion recess, and the tip of the side hole needle may be inserted into the slit. In short, the cover valve body may be provided with one slit penetrating in the thickness direction. Furthermore, in the preceding first to fourth practical embodiments, the slit 78, 173 is provided in addition to the insertion recess 76, 172, but the slit 78, 173 need not be provided. That is, when the cover valve body moves to the proximal end side with respect to the side hole needle at the time of connection with the female connector, the tip of the side hole needle may break through the cover valve body and protrude from the cover valve body to the tip side. By adopting such an embodiment, exposure or leakage of a fluid such as a drug solution from the male connector before connection with the female connector can be more reliably prevented.

Further, in the preceding first to fourth practical embodiments, the coil spring 82, 186 is adopted as an urging means for urging the restraining member 56, 150, 224 and the cover valve body 74, 170, 222 toward the tip side. However, the present invention is not limited to this embodiment. That is, instead of the coil spring, a tubular body or the like made of an elastic material may be arranged between the female luer portion of the male connector and the restraining member in the axial direction, or any conventionally known urging means can be adopted including using a magnetic force other than the elasticity. When the urging means is made of an elastic material such as rubber, it may be integrally formed with the cover valve body.

Further, the female connector connected to the male connector 10, 120, 220, 270 in the preceding first to fourth practical embodiments is not limited to those shown in FIGS. 5 and 15. For example, the female connector 86, 190 in the first to fourth practical embodiments is provided with the valve member 94, 198, but the valve member is not essential. Further, in the female connector 86, 190, the housing that supports the valve member 94, 198 is constituted by the base member 88, 192, the lower cover member 90, 194, and the upper cover member 92, 196. However, the housing is not limited to such a divided structure, but may be constituted by a single member.

The restraining member may directly cover the cover valve body, but for example, an elastic member separate from the cover valve body may be interposed between the cover valve body and the restraining member.

Further, for example, in the preceding first practical embodiment, the cover valve body 74 and the restraining member 56 are provided as members into which the side hole needle 14 is inserted. When the male connector 10 and the female connector 86 are connected, the upper cover member 92 of the female connector 86 comes into contact with the restraining member 56 via the cover valve body 74, so that the cover valve body 74 and the restraining member 56 will not be inserted in the female connector 86. However, it would also be acceptable that by adjusting the relative diametrical dimensions between the female connector 86, and the cover valve body 74 and the restraining member 56, the cover valve body 74 and the restraining member 56, which are members into which the side hole needle 14 is inserted, may function as a male luer and be inserted into the female connector to realize connection communication of a fluid flow path. For example, the tip of the cover valve body of the male connector may press the base end side end surface of the valve member of the female connector, so that the base end side end surface of the valve member of the female connector and the tip side end surface of the cover valve body of the male connector form a liquid-tight sealing surface in a state where the valve member of the female connector is open.

Furthermore, the engagement between the restraining member and the male connector housing does not have to be a concave-convex fitting. For example, in the preceding second and fourth practical embodiments, it would also be acceptable that the outer peripheral surface of the outer tubular portion is an annular surface without any concave or convex portion, and in the initial state, the fitting convex portions 148, 148 sandwich the outer tubular portion in the vertical direction by a frictional force or the like due to contact with the outer peripheral surface of the outer tubular portion, thereby engaging the restraining member and the male connector housing.

Further, in the preceding second and fourth practical embodiments, the operation portion is configured such that the operation portion can be lightly operated by the operation piece 188 protruding toward the proximal end side by utilizing the action of the lever. However, the engagement between the male connector housing and the restraining member may be released by pulling an operation portion formed so as to project from the fitting convex portion 148 to the outer peripheral side. Further, as in the preceding second and fourth practical embodiments, the male connector housing and the engagement mechanism are fixedly provided to the side hole needle by integral molding or fixing of a separate member, thereby simplifying the structure or the like. However, as another embodiment, it would also be possible to constitute the engagement mechanism with a separate member that is not fixed to the side hole needle or the male connector housing. For example, it would also be acceptable to attach a separate engagement pin so as to penetrate the outer tubular portion 136 of the male connector housing 132 inward and outward, and the tip portion of the engagement pin protruding to the inner peripheral side may be engaged with the fitting concave portion 168 of the restraining member 150, while the engagement may be released by the portion of the engagement pin protruding to the outer peripheral side being pulled outward. Further, instead of providing a fitting protrusion that engages with the restraining member integrally with the operation member (operation piece 188), it would also be acceptable, for example, to provide a fitting protrusion to a detachable member such as a cap that is detachably attached to the restraining member 150 or the male connector housing 132, and the cover valve body may be allowed to move in the axial direction by removing the detachable member.

Further, the engaging portion with the restraining member does not need to be integrally provided with the operation piece. For example, it would also be possible to provide a pressing member that presses the tip protruding portion 151 of the restraining member 150 inward in the radial direction as a member separate from the restraining member 150 and the operation piece 188, and by the pressing member being pressed and moved inward in the radial direction by the operation piece 188 to deform the tip protruding portion 151 of the restraining member 150 inward in the radial direction, the engagement of the fitting convex portion 148 with respect to the restraining member 150 may be released, thereby allowing the restraining member 150 to move in the axial direction.

Further, the engaging portion between the male connector housing and the restraining member, as well as the operation portion for disengaging the said engagement are not limited to the embodiment in which a pair of upper and lower parts thereof are provided. For example, the fitting concave portion may be provided at one place on the circumference, or may be provided at three or more places, so as to substantially correspond to the position of the fitting convex portion.

Further, in the male connector housing 132 in the preceding second and fourth practical embodiments, the engaging portion with the restraining member 150 and the engaging portion with the female connector 190 are both constituted by the fitting convex portion 148, but the engaging portion with the restraining member and the engaging portion with the female connector may be constituted by mutually different members. For example, in the male connector housing, an engaging portion with the female connector may be provided at a portion different from the fitting convex portion 148 in the circumferential direction (for example, at the high-rigidity wall portion 146) or at a portion different from the fitting convex portion 148 in the axial direction.

Further, in the preceding second and fourth practical embodiments, the female connector may be connected to the male connector by screw feed. For example, a female screw portion may be provided on the inner peripheral surface of the male connector housing, while a male screw portion may be provided on the outer peripheral surface of the female connector, so that the male connector housing and the female connector are engaged by screwing the male screw portion and the female screw portion. Furthermore, the restraining member may also be moved with respect to the male connector housing by screw feed, and it is acceptable as long as the engagement between the male connector housing and the restraining member are released by the release mechanism before the restraining member moves. In the preceding fourth practical embodiment, the restraining member 150 is provided with the female screw portion 272 that is screwed with the male screw portion 208 of the female connector 190. However, for example, it would also be possible to provide a portion that protrudes from the male connector housing to the tip side, and to provide a female screw portion that is screwed with a male screw portion of the female connector on the inner peripheral surface of the protruding portion.

In the preceding second practical embodiment, a tubular member (adapter) may be externally mounted on the small-diameter tubular portion of the female connector. In that case, the outer diameter dimension of the adapter is made substantially equal to the inner diameter dimension of the large-diameter recess of the restraining member, and the outer peripheral surface of the adapter and the inner peripheral surface of the large-diameter recess may guide the insertion of the female connector into the large-diameter recess. Alternatively, a female screw portion may be provided on the inner peripheral surface of the large-diameter recess and a male screw portion may be provided on the outer peripheral surface of the adapter, so that the restraining member and the female connector are connected by screwing the male screw portion and the female screw portion. Alternatively, the adapter may have a structure that covers as far as the large-diameter tubular portion of the female connector, so that, for example, the female screw portion provided on the inner peripheral surface of the male connector housing and the male screw portion provided on the outer peripheral surface of the adapter are screwed together.

Further, in the preceding second and fourth practical embodiments, the flange-shaped portion 210 projecting to the outer peripheral side is provided on the outer peripheral surface of the female connector 190, and is engaged with the fitting convex portion 148 of the male connector housing 132. However, the engagement mechanism between the female connector 190 and the male connector 120 is not limited as long as it limits mutual axial movement by engagement. For example, it would also be acceptable to provide an engaging recess that opens on the outer peripheral surface of the housing of the female connector 190 partially in the circumferential direction or continuously over the entire circumference, and the fitting convex portion 148 of the male connector 120 is engaged with the engaging recess.

Further, in the preceding second and fourth practical embodiments, the tip protruding portion 151 of the restraining member 150 protrudes toward the tip side with respect to the cover valve body 170, but the present invention is not limited to this embodiment. The cover valve body may be located on the tip side with respect to the restraining member and exposed to the outside, or the tip protruding portion may protrude partially in the circumferential direction to be arranged on the outer peripheral side of the cover valve body, other than being tubular or annular as in the second practical embodiment. Further, the member for determining the flow path on the base end side of the cover valve body is not limited to the side hole needle, but may be a needle having a puncture end or a tubular body such as a luer.

In the preceding first to fourth practical embodiments, the side hole needle 14, 124 is a separate member from the needle hub portion 34, 130 (that is, the male connector housing 18, 132) and the needle hub 230, and is attached later, but may be integrally formed. For example, the side hole needle and the needle hub portion (that is, the male connector housing) or the needle hub may be constituted by an integrally molded product made of a hard synthetic resin. As a result, the number of parts can be reduced, and operations such as later attachment can be omitted.

The size of the opening with respect to the side hole needle is not limited, but for example, the flow path cross-sectional area of the opening is preferably 70% or less of the flow path cross-sectional area of the inner hole of the side hole needle, and more preferably is set within the range of 40% to 60%. In short, the inner diameter dimension øA of the opening (see FIG. 18) is preferably 85% or less of the inner diameter dimension øB of the inner hole (see FIG. 18), for example, and more preferably is set within the range of 60% to 80%. When such openings having a relatively small diameter are provided, it is preferable that a plurality of openings are provided on the same circumference. For example, three to six lateral openings may be provided in the side hole needle. By setting the number and the size of the openings of the side hole needle as described above, the exposure of the drug solution can be effectively prevented, and the flow rate of the drug solution flowing through the side hole needle can be sufficiently obtained. In particular, by reducing the axial dimension of the opening, the time for passing between the cover valve body of the male connector and the valve member of the female connector can be shortened, thereby reducing the risk of leakage of the drug solution into a gap therebetween.

Further, in the preceding first to fourth practical embodiments, the side hole needle 14, 124 has inner and outer diameters that are substantially constant in the axial direction and extends substantially straight, but the inner and outer diameters of the tip of the side hole needle may be smaller than those of the proximal end portion of the side hole needle. It is preferable that such a small diameter portion is formed with a certain axial length from the tip of the side hole needle. For example, only the portion to be inserted through the cover member and the valve member of the female connector when connected to the female connector may have a small diameter. As a result, the flow efficiency of the fluid in the portion having a small diameter is reduced, and the generation of a gap is avoided between the side hole needle and a combination of the cover member and the valve member at the time of insertion of the needle through the cover member and the valve member, thereby preventing leakage or exposure of the drug solution through the said gap. Further, by the tip of the side hole needle having a small diameter, the effects of improving the insertability and reducing the frictional resistance are exhibited, and the deformation of the cover member and the valve member is suppressed. This also exhibits the effects of preventing the generation of a gap as well as preventing the leakage and exposure of the drug solution through the said gap.

The slit and the insertion recess provided in the cover valve body may be a pin hole having a diameter smaller than the outer diameter dimension of the side hole needle. As a result, the gap between the side hole needle and the cover valve body is made smaller in comparison with the case of the slit, thereby reducing the risk of leakage or exposure of the drug solution.

Further, the base member of the male connector and the valve member of the female connector may be formed with mutually different hardness (flexibility). For example, the base member of the male connector may be formed more flexibly than the valve member of the female connector. As a result, when the base member and the valve member are deformed in an overlapped state, the base member can be deformed following the valve member, thereby reducing the risk of generation of a gap between the base member and the valve member.

### KEYS TO SYMBOLS

10: male connector, 12: opening, 14: side hole needle (hollow needle), 16: inner hole, 17: tip surface, 18: male connector housing, 20: female luer portion, 22: luer peripheral wall portion, 24: female luer taper surface, 26: base end side opening portion, 28: male screw portion, 30: bottom wall portion, 32: through hole, 33: flat surface, 34: needle hub portion, 36: outer tubular portion, 38: notch, 40: opening window, 42: lock portion, 44: lock peripheral wall portion, 46: upper bottom wall portion, 48: through hole, 49: flat surface, 50: female screw portion, 52: ridge, 54: flat surface, 56: restraining member, 58: stepped surface, 60: outer wall portion, 62: locking claw, 64: bottom wall portion, 66: through hole, 68: proximal end side protrusion, 70: engagement claw, 72: notch, 74: cover valve body, 76: insertion recess, 78: slit, 80: locking recess, 82: coil spring (urging means), 84: restricting means, 86: female connector, 88: base member, 90: lower cover member, 92: upper cover member, 94: valve member, 96: female luer portion, 98: slit, 100: stepped surface, 101: small-diameter tubular portion, 102: male screw portion, 104: tubular portion, 106: fluid flow path , 108: outer peripheral surface (guide portion), 110: inner peripheral surface, 112: positioning means, 120: male connector, 122: opening, 124: side hole needle (hollow needle, tubular body), 126: inner hole, 128 : tip surface, 130: needle hub portion, 132: male connector housing, 134: female luer portion, 135: tapered surface, 136: outer tubular portion, 138: bottom plate portion, 140: proximal end wall portion, 142: insertion hole, 143: notch, 144: flexible wall portion, 146: wall portion, 148: fitting convex portion (movement restriction mechanism, engagement mechanism, connection engaging portion), 150: restraining member (holding member), 151: tip protruding portion, 152: large-diameter recess, 154: tubular portion (outer peripheral wall portion), 156: inner peripheral protrusion (bottom wall portion), 158: accommodating area, 160: proximal end side protrusion, 162: notch, 164: leg portion, 166: positioning recess, 168: fitting concave portion (movement restriction mechanism, engagement mechanism), 170: cover valve body, 172: insertion recess, 173: slit, 174: tip surface, 174a: central portion, 174b: outer peripheral portion, 175: tubular outer peripheral surface, 175a: stepped surface, 175b: tip side outer peripheral surface, 175c: base end side outer peripheral surface, 176: annular member, 177: pressing protrusion, 178: lid member, 180: positioning protrusion, 182: positioning convex portion, 184: guide window, 186: coil spring (urging means), 187: positioning means, 188: operation piece (engagement mechanism, operation portion), 190: female connector, 192: base member, 194: lower cover member, 196: upper cover member, 198: disc valve (valve member), 198a: central portion, 198b: support portion, 200: female luer portion, 202: slit, 204: stepped surface, 206: small-diameter tubular portion, 208: male screw portion, 210: flange-shaped portion, 212: large-diameter tubular portion, 216: fluid flow path, 220: male connector, 222: cover valve body, 224: restraining member, 226: male connector housing, 228: tubular peripheral wall portion, 230: needle hub, 232: through window, 234: positioning recess, 236: tapered surface, 238: inner peripheral groove portion, 240: inner peripheral protrusion, 242: female screw portion, 244: female luer portion, 246: needle hub portion, 248: annular recess, 250: engaging protrusion, 252: positioning convex portion, 254: tapered surface, 256: pressing protrusion, 258: outer peripheral protrusion, 270: male connector, 272: female screw portion (connecting portion), 280: male connector, 282: female luer portion, 284: lock portion, 286: tubular body, 288: ratchet mechanism, 290: male connector, 292: female luer portion, 294: lock portion, 296: ratchet mechanism, 300: male connector, 302: cover valve body, 304: O- ring (sealing portion), 306: limiting member, 310: male connector, 312: male connector housing, 314: tubular peripheral wall portion, 316: cover portion, 318: cover valve body, 320: connecting wall portion, 330: cover valve body, 332: tip side slit, 334: base end side slit

## Claims

1. A male connector comprising:
a side hole needle having a hollow needle with an inner hole and an opening through which the inner hole is opened sideways at a tip of the hollow needle, the side hole needle being configured to be inserted into a female connector as a connection partner so as to connect a fluid flow path thereof with that of the female connector;
an elastic cover valve body provided so as to be movable in an axial direction with respect to the side hole needle, the elastic cover valve body being configured to cover the opening of the inner hole at a tip of the side hole needle;
a positioning means for elastically positioning the cover valve body to the tip of the side hole needle while allowing the cover valve body to move to a proximal end side of the side hole needle; and
a rigid restraining member that covers an outer periphery of the cover valve body.

2. The male connector according to claim 1, wherein the restraining member includes: a proximal end side protrusion extending toward a proximal end side of the side hole needle; and a guide portion provided at the proximal end side protrusion to guide an axial movement of the restraining member with respect to a male connector housing.

3. The male connector according to claim 1 or 2, further comprising a male connector housing having a tubular peripheral wall portion that covers an outer periphery of the restraining member.

4. The male connector according to claim 3, wherein the tubular peripheral wall portion has a structure separate from a needle hub of the side hole needle.

5. The male connector according to claim 3 or 4, wherein the tubular peripheral wall portion has a tubular cover portion protruding toward a tip side of the side hole needle and having a diameter larger than that of the tubular peripheral wall portion.

6. The male connector according to any one of claims 1-5, further comprising a connecting portion configured to be detachably connected to the female connector with the cover valve body being abutted and superposed on a valve member of the female connector, wherein the side hole needle is insert-able and removable from the cover valve body and the valve member of the female connector under a contact state of the cover valve body with the valve member of the female connector by the connecting portion.

7. A male connector comprising:
a tubular body having an inner hole and an opening through which the inner hole is opened at a tip of the tubular body, the tip of the tubular body being configured to be inserted into a female connector as a connection partner so as to connect a fluid flow path thereof with that of the female connector;
an elastic cover valve body configured to cover the opening of the tip of the tubular body;
a holding member holding the cover valve body and being movable in an axial direction of the tubular body; and
a releasable movement restriction mechanism for restricting a movement of the holding member holding the cover valve body in the axial direction of the tubular body so as to hold the opening of the tip of the tubular body in a sealed state by the cover valve body.

8. A male connector comprising:
a tubular body having an inner hole and an opening through which the inner hole is opened at a tip of the tubular body; and
an elastic cover valve body configured to cover the opening of the tip of the tubular body, wherein
the tip of the tubular body is configured to be projected out from the cover valve body to be inserted into a female connector as a connection partner so as to connect a fluid flow path thereof with that of the female connector,
a tip surface of the cover valve body has a centrally protruding shape that protrude axially outwardly from an outer periphery to a central,
a central portion of the tip surface of the cover valve body is a curved tip surface that is convex toward the tip, and
a region that spreads out from the central portion toward the outer periphery is an inverted curved inclined surface that becomes concave toward the tip.

9. A male connector comprising:
a side hole needle having a hollow needle with an inner hole and an opening through which the inner hole is opened sideways at a tip of the hollow needle, the side hole needle being configured to be inserted into a female connector as a connection partner so as to connect a fluid flow path thereof with that of the female connector;
an elastic cover valve body provided so as to be movable in an axial direction with respect to the side hole needle, the elastic cover valve body being configured to cover the opening of the inner hole at a tip of the side hole needle; and
a pin hole provided on a central axis of the cover valve body on which the side hole needle is inserted, the pin hole having a diameter at least at a base end side smaller than that of the side hole needle.

10. A male connector comprising:
a side hole needle having a hollow needle with an inner hole and an opening through which the inner hole is opened sideways at a tip of the hollow needle, the side hole needle being configured to be inserted into a female connector as a connection partner so as to connect a fluid flow path thereof with that of the female connector;
an elastic cover valve body provided so as to be movable in an axial direction with respect to the side hole needle, the elastic cover valve body being configured to cover the opening of the inner hole at a tip of the side hole needle;
an urging means for urging the cover valve body from a base end side toward a tip side where the cover valve body covers the opening of the inner hole of the side hole needle; and
a limiting member that is superposed on a base end surface of the cover valve body and limits deformation of the base end surface.

11. A male connector comprising:
a side hole needle having a hollow needle with an inner hole and an opening through which the inner hole is opened sideways at a tip of the hollow needle, the side hole needle being configured to be inserted into a female connector as a connection partner so as to connect a fluid flow path thereof with that of the female connector;
an elastic cover valve body provided so as to be movable in an axial direction with respect to the side hole needle, the elastic cover valve body being configured to cover the opening of the inner hole at a tip of the side hole needle; and
an annular sealing portion for sealing between the cover valve body and an outer peripheral surface of the side hole needle, the annular sealing portion being provided on a base end side of the cover valve body.

12. A male connector comprising:
a side hole needle having a hollow needle with an inner hole and a plurality of openings through which the inner hole is opened sideways at a tip of the hollow needle, the side hole needle being configured to be inserted into a female connector as a connection partner so as to connect a fluid flow path thereof with that of the female connector; and
an elastic cover valve body provided so as to be movable in an axial direction with respect to the side hole needle, the elastic cover valve body being configured to cover the opening of the inner hole at a tip of the side hole needle, wherein
the plurality of openings are formed on a same circumference, and have a flow path cross-sectional area of 70% or less of that of the inner hole of the side hole needle.

13. A male connector comprising:
a side hole needle having a hollow needle with an inner hole and an opening through which the inner hole is opened sideways at a tip of the hollow needle, the side hole needle being configured to be inserted into a female connector as a connection partner so as to connect a fluid flow path thereof with that of the female connector; and
an elastic cover valve body provided so as to be movable in an axial direction with respect to the side hole needle, the elastic cover valve body being configured to cover the opening of the inner hole at a tip of the side hole needle, wherein
inner and outer diameters of the tip of the side hole needle inserted into the cover valve body are smaller than those of a proximal end portion of the side hole needle.

14. A male connector comprising:
a side hole needle having a hollow needle with an inner hole and an opening through which the inner hole is opened sideways at a tip of the hollow needle, the side hole needle being configured to be inserted into a female connector as a connection partner so as to connect a fluid flow path thereof with that of the female connector; and
an elastic cover valve body provided so as to be movable in an axial direction with respect to the side hole needle, the elastic cover valve body being configured to cover the opening of the inner hole at a tip of the side hole needle, wherein
the cover valve body is formed more flexibly than a valve member of the female connector.
